# EUROPEAN PATENT APPLICATION

(11) **EP 2 645 109 A2**
(43) Date of publication of application: **02.10.2013**
(21) Application number: 13158536.6
(22) Date of filing: 11.03.2013
(51) Int. Cl.: G01N 35/00

(54) **Sample processing apparatus, sample processing system and sample processing method**

(30) Priority: 30.03.2012 JP 2012080318
(71) Applicant: SYSMEX CORPORATION, Kobe-shi, Hyogo 651-0073 (JP)
(72) Inventor: Tatsutani, Hiroo, Hyogo, 651-0073 (JP)
(74) Representative: HOFFMANN EITLE

(57) **Abstract**

The present invention is a sample processing apparatus. The sample processing apparatus includes: a reader configured to read, from a storage medium attached to a sample container containing a sample or a sample rack supporting the sample container, an analysis value which has been generated by a sample analyzer analyzing the sample for a predetermined analysis item; a sample processing section which comprises an operating mechanism configured to perform an operation for processing the sample, and a controller configured to control the operating mechanism of the sample processing section so as to perform processing on the sample based on the analysis value read by the reader.

## Description

### FIELD OF THE INVENTION

The present invention relates to sample processing apparatuses, and sample processing systems which process clinical samples such as blood and urine, and sample processing methods therefor.

### BACKGROUND

Conventionally, there are known sample processing apparatuses which perform sample processing such as retesting, smear preparation, and the like, based on a result of a sample analysis previously performed. Japanese Laid-Open Patent Publication No. S62-226058 proposes a sample analyzing system in which automated sample analysis is performed by using a cassette from/to which information can be read/written through radio waves. In this system, a sample container can be accommodated in a cassette on which analysis items and the destination of the sample (analyzer number) are written. When a cassette accommodating a sample container is placed on an unmanned transportation car, the unmanned transportation car reads the analyzer number being the destination from the cassette, and transports the cassette along with the sample container to its corresponding analyzer. Then, in the analyzer, analysis items are read as appropriate from the cassette, and desired analysis is performed on the sample contained in the sample container. Then, after the sample analysis has been performed, its analysis result is transmitted to a system computer connected to the analyzer, and this computer determines whether retesting is necessary or not based on the received analysis result. When retesting is necessary, retesting information is written on the cassette. As a result, the unmanned transportation car moves to the corresponding analyzer, and sample analysis (retesting) is performed in this analyzer.

However, in the sample analyzing system described in Japanese Laid-Open Patent Publication No. S62-226058, when sample analysis is performed in an analyzer, it is necessary to send the analysis result to a system computer, which may cause complicated communication processing. In addition, since the system computer determines whether retesting is necessary based on the analysis result and generates retesting information, in the case where a new analyzer is added in the sample analyzing system or where an analyzer included in the sample analyzing system is replaced with another apparatus, it is necessary to change the setting of the system computer, accordingly. Thus, a lot of time and cost may be spent.

In view of the above problems, an object of the present invention is to enable performance of sample processing such as retesting and smear preparation, without transmitting a result of sample analysis performed by a sample analyzer to another computer, based on the result of sample analysis which has been previously performed by the sample analyzer.

### SUMMARY

The scope of the present invention is defined solely by the appended claims, and is not affected to any degree by the statements within this summary.

A first aspect of the present invention is a sample processing apparatus comprising: a reader configured to read, from a storage medium attached to a sample container containing a sample or a sample rack supporting the sample container, an analysis value which has been generated by a sample analyzer analyzing the sample for a predetermined analysis item; a sample processing section which comprises an operating mechanism configured to perform an operation for processing the sample, and a controller configured to control the operating mechanism of the sample processing section so as to perform processing on the sample based on the analysis value read by the reader.

According to the sample processing apparatus of the present aspect, since an analysis value obtained by the sample analyzer is stored in the storage medium, it is possible to determine the content of sample processing based on the analysis value read by the reader. Therefore, without transmitting the result of the sample analysis performed by the sample analyzer to another computer, the sample processing apparatus can perform sample processing such as retesting and smear preparation, based on the result of the sample analysis which has been previously performed by the sample analyzer. Accordingly, it is possible to suppress communication processing in the sample processing apparatus from being complicated. Further, since another computer need not determine a content of sample processing based on the result of the sample analysis, it is possible to save time and costs necessary for setting the another computer.

Preferably, the sample processing section is a sample analyzing section configured to analyze the sample, and the controller controls the operating mechanism of the sample analyzing section so as to analyze the sample for an analysis item according to the analysis value read by the reader.

Preferably, the sample processing apparatus further comprises a writer configured to write a result of a sample analysis performed by the sample analyzing section into the storage medium. Accordingly, another sample processing apparatus can use the result of the sample analysis. Therefore, another sample processing apparatus can smoothly perform processing.

Preferably, the sample processing section is a smear preparation section configured to prepare a smear by smearing a sample, and when the analysis value read by the reader satisfies a predetermined condition, the controller controls the operating mechanism so as to prepare the smear.

Preferably, the controller controls the operating mechanism of the smear preparation section so as to prepare the smear on a condition according to the analysis value read by the reader. Accordingly, the sample processing apparatus need not communicate with another apparatus in order to determine a smear preparation condition.

Preferably, the sample is a biospecimen collected from a subject.

Preferably, the sample processing apparatus further comprises:the sample analyzer; a writer configured to write the analysis value generated by the sample analyzer into the storage medium; and a transporting apparatus configured to transport the sample container or the sample rack to the sample processing section, the analysis value having been written by the writer into the storage medium attached to the sample container or the sample rack, wherein the reader reads the analysis value from the storage medium attached to the sample container or the sample rack transported by the transporting apparatus.

Preferably, the storage medium is attached to the sample rack, the sample rack is capable of supporting a plurality of sample containers, the sample processing apparatus further comprises a detector configured to detect a support position of each sample container in the sample rack, and the writer writes, into the storage medium, an analysis value of a sample in each sample container in association with a support position of each sample container detected by the detector, respectively. Accordingly, analysis results of the respective samples can be read at one time from the sample rack, and support positions of the respective samples can be identified. Therefore, a sample processing apparatus that has received the sample rack can smoothly perform processing on the samples based on their respective analysis results.

Preferably, the transporting apparatus is formed by a first transporting unit corresponding to the sample analyzer and a second transporting unit corresponding to the sample processing section, and the reader is arranged in the second transporting unit, and reads the analysis value from the storage medium attached to the sample container or the sample rack that has been transported from the first transporting unit to the second transporting unit.

Preferably, the sample processing apparatus further comprises a second sample processing section comprising an operating mechanism configured to perform an operation for processing the sample transported by the transporting apparatus, wherein the controller determines, from among the sample processing section and the second sample processing section, a transportation destination for the sample analyzed by the sample analyzer, based on the analysis value read by the reader, and controls the transporting apparatus so as to transport the sample to the determined transportation destination.

Preferably, the storage medium is an electronic tag communicable with the reader, and the reader is configured to read the analysis value from the electronic tag, by using radio waves.

A second aspect of the present invention is a sample processing system comprising: a sample analyzing section configured to analyze a sample to generate an analysis value for a predetermined analysis item; a transporting apparatus configured to transport a sample container containing the sample analyzed by the sample analyzing section or a sample rack supporting the sample container; a first to an nth sample processing sections each comprising an operating mechanism configured to perform an operation for processing the sample transported by the transporting apparatus, and arranged downstream to the sample analyzing section in a sample transporting direction of the transporting apparatus; a writer configured to write the analysis value generated by the sample analyzing section into a storage medium attached to the sample container containing the sample analyzed by the sample analyzing section or the sample rack supporting the sample container; a reader configured to read the analysis value written by the writer from the storage medium; and a transportation controller configured to determine, from among the first to the nth sample processing sections, a transportation destination for the sample analyzed by the sample analyzing section, based on the analysis value read by the reader, and configured to control a transporting operation of the transporting apparatus so as to transport the sample to the determined transportation destination.

According to the sample processing system of the present aspect, a transportation destination for the sample analyzed by the sample analyzing section is determined from among the first to the nth sample processing sections based on the analysis value read from the storage medium, and the sample is transported to the determined transportation destination. Accordingly, the sample analyzed by the sample analyzing section can be efficiently transported to the corresponding one of the first to the nth sample processing sections arranged on the downstream side.

Preferably, the transportation controller stores a rule set for determining whether it is necessary to perform sample processing in each of the first to the nth sample processing sections, and determines the transportation destination for the sample, based on the analysis value read by the reader and the rule set. Accordingly, the transportation controller can assuredly determine a transportation destination for a sample based on the rule set.

Preferably, the sample processing system further comprises a sample collection section configured to collect a sample for which processing has been completed, wherein when it is not necessary to perform processing on the sample in any of the first to the nth sample processing sections, the transportation controller controls the transporting operation of the transporting apparatus so as to transport the sample to the sample collection section. Accordingly, since a sample that need not be subjected to processing in any of the first to the nth sample processing sections is transported to the sample collection section, the transportation controller can cause a sample that needs to be subjected to processing in the first to the nth sample processing sections to be more quickly transported.

A third aspect of the present invention is a sample processing method to be used in a sample processing system which includes a sample analyzer and at least one sample processing apparatus, the method comprising: writing an analysis value generated by the sample analyzer analyzing a sample for a predetermined analysis item, into a storage medium attached to a sample container containing the sample or a sample rack supporting the sample container; reading the analysis value from the storage medium in the sample processing apparatus; and performing processing on the sample by using the sample processing apparatus, based on the read analysis value.

According to the sample processing method of the present aspect, an analysis value obtained by the sample analyzer is written in the storage medium, the analysis value is read from the storage medium in the sample processing apparatus, and based on the read analysis value, the sample processing apparatus determines a content of sample processing. Therefore, without transmitting the result of the sample analysis performed by the sample analyzer to another computer, the sample processing apparatus can perform sample processing such as retesting and smear preparation, based on the result of the sample analysis which has been previously performed by the sample analyzer. Therefore, it is possible to suppress communication processing in the sample processing apparatus from being complicated. Further, since another computer need not determine a content of sample processing based on the result of the sample analysis, it is possible to save time and costs necessary for setting the another computer.

### BRIEF DESCRIPTION OF THE DRAWINGS

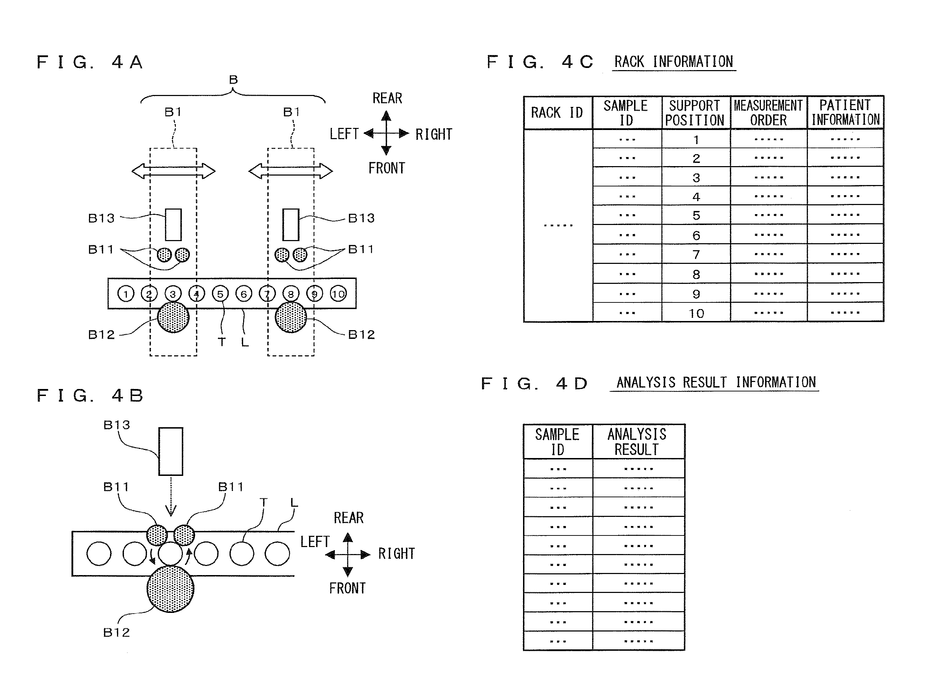

FIG. 1 is a schematic diagram showing a configuration of a sample processing system according to Embodiment 1 viewed from above;
FIG. 2A shows a configuration of a sample container according to Embodiment 1, and FIG. 2B shows a configuration of a sample rack according to Embodiment 1;
FIG. 3 shows configurations of a loading unit, a preprocessing unit, and a collection unit according to Embodiment 1 viewed from above;
Each of FIG. 4A and FIG. 4B is a schematic diagram showing a configuration of a bar code unit according to Embodiment 1 viewed from above, FIG. 4C is a schematic diagram showing a configuration of rack information, and FIG. 4D is a schematic diagram showing a configuration of analysis result information;

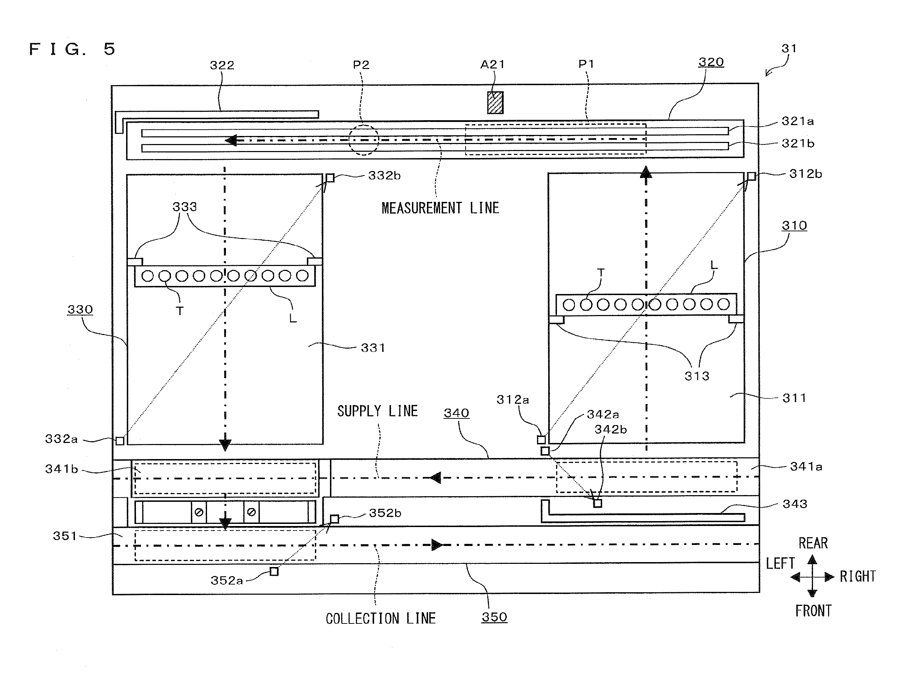

FIG. 5 shows a configuration of a transporting unit according to Embodiment 1 viewed from above;

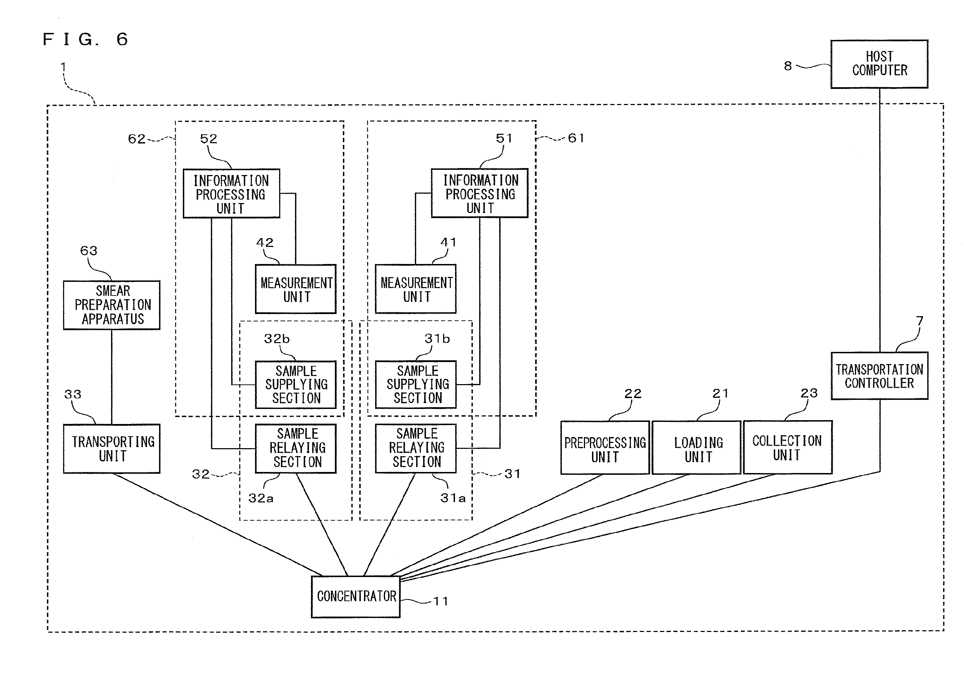

FIG. 6 shows connection relationship between units and apparatuses of the sample processing system according to Embodiment 1;

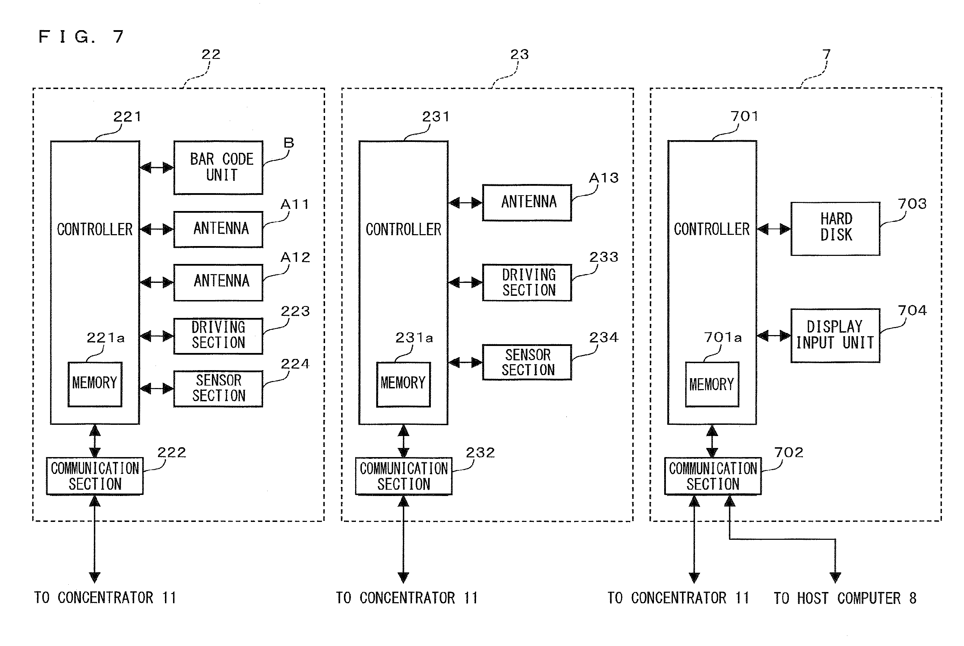

FIG. 7 is a schematic diagram showing configurations of the preprocessing unit, the collection unit, and a transportation controller according to Embodiment 1;

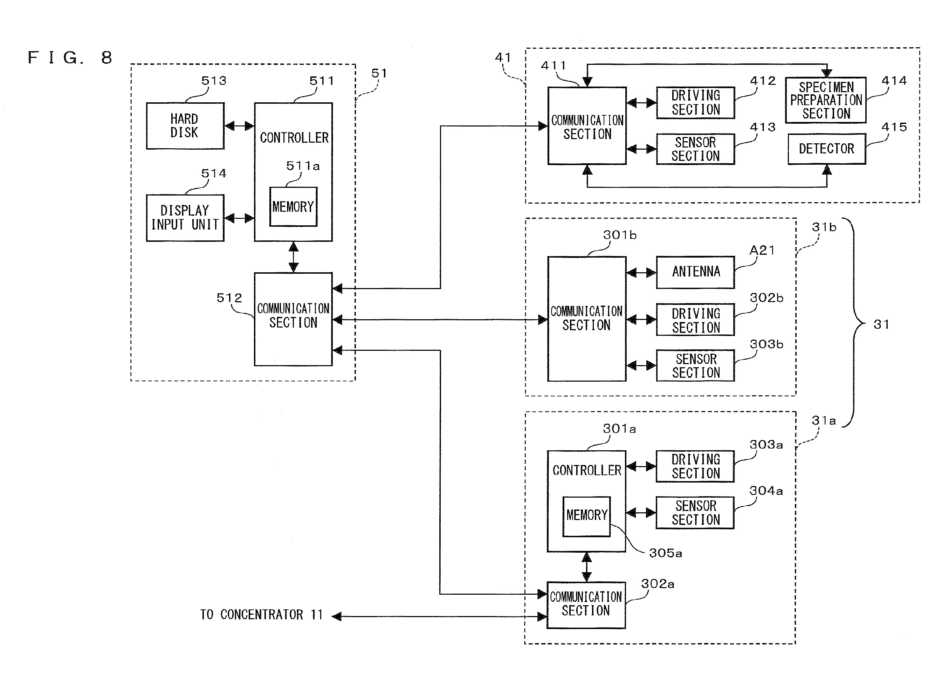

FIG. 8 is a schematic diagram showing configurations of a transporting unit, a measurement unit, and an information processing unit according to Embodiment 1;

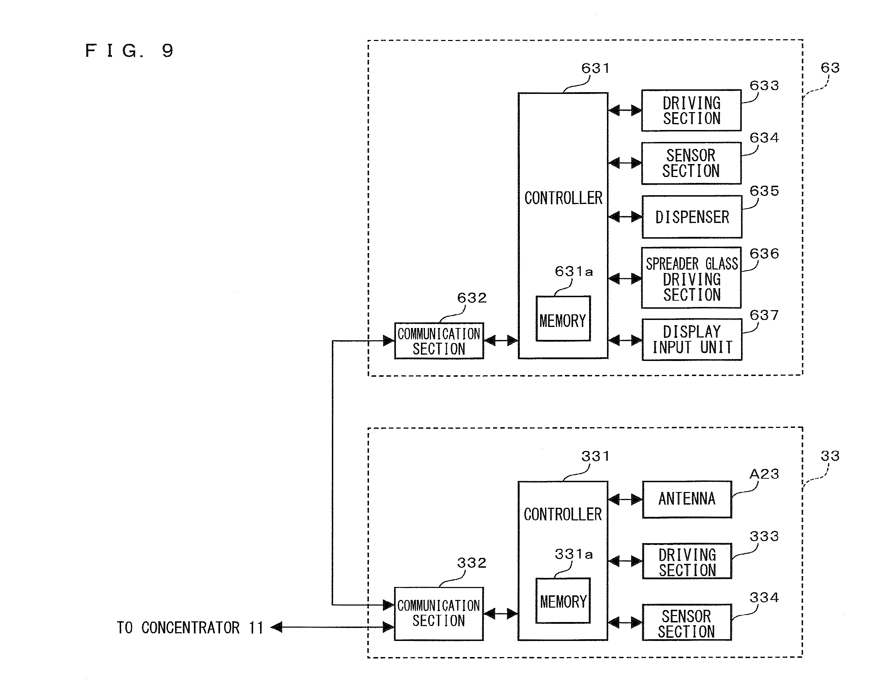

FIG. 9 is a schematic diagram showing configurations of a transporting unit and a smear preparation apparatus according to Embodiment 1;

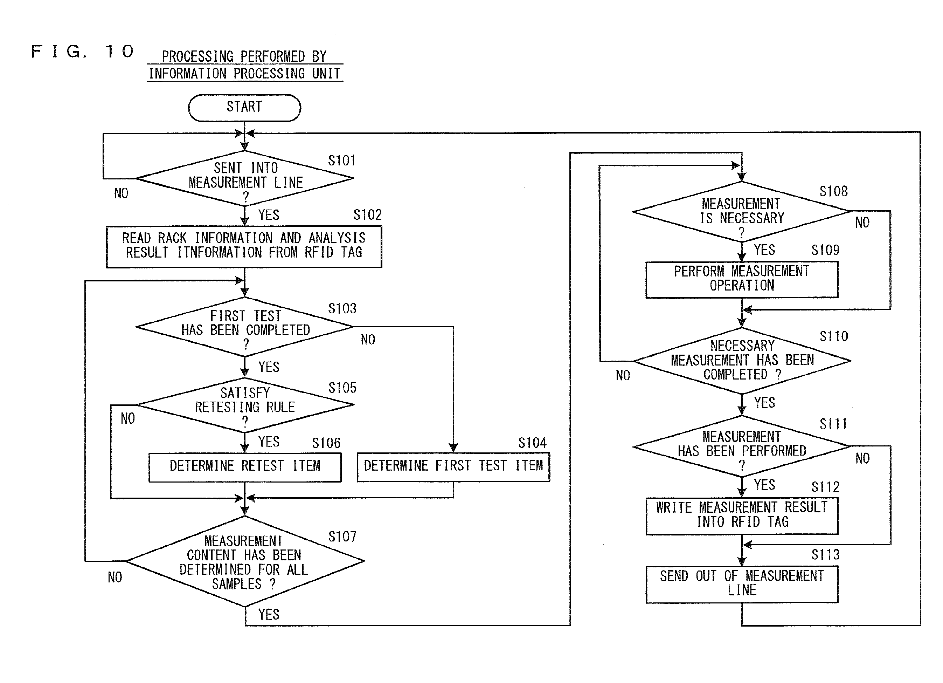

FIG. 10 is a flow chart showing processing performed by an information processing unit according to Embodiment 1;

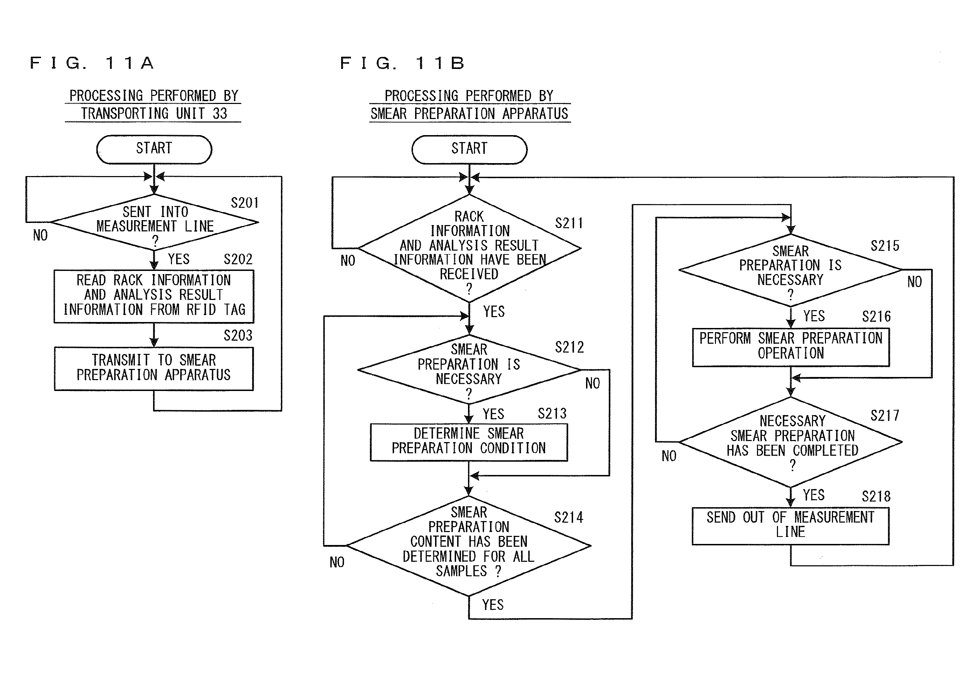

FIG. 11A is a flow chart showing processing performed by a transporting unit according to Embodiment 1, and FIG. 11B is a flow chart showing processing performed by the smear preparation apparatus according to Embodiment 1;

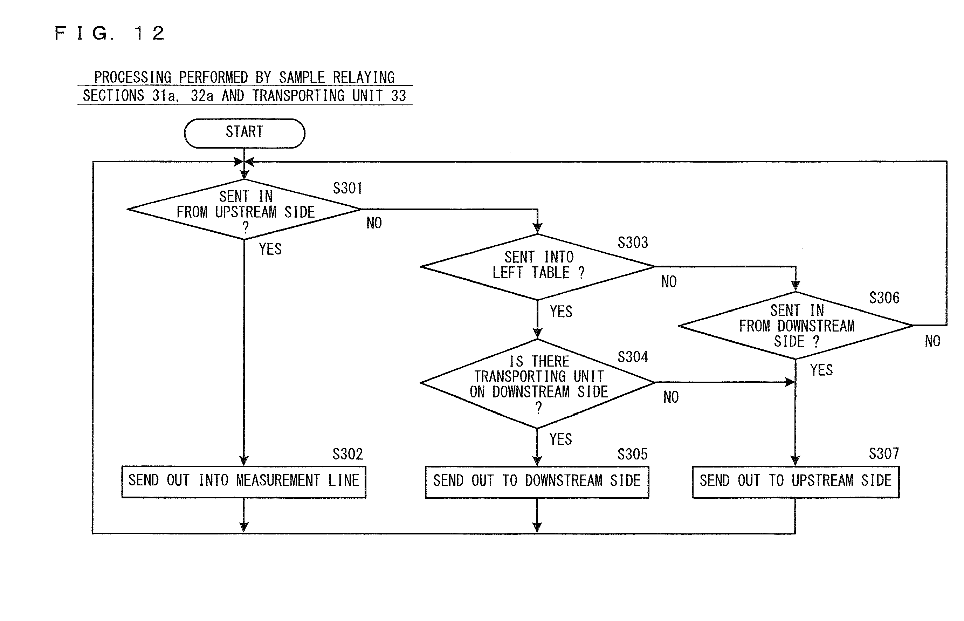

FIG. 12 is a flow chart showing processing performed by a transporting unit according to Embodiment 1;

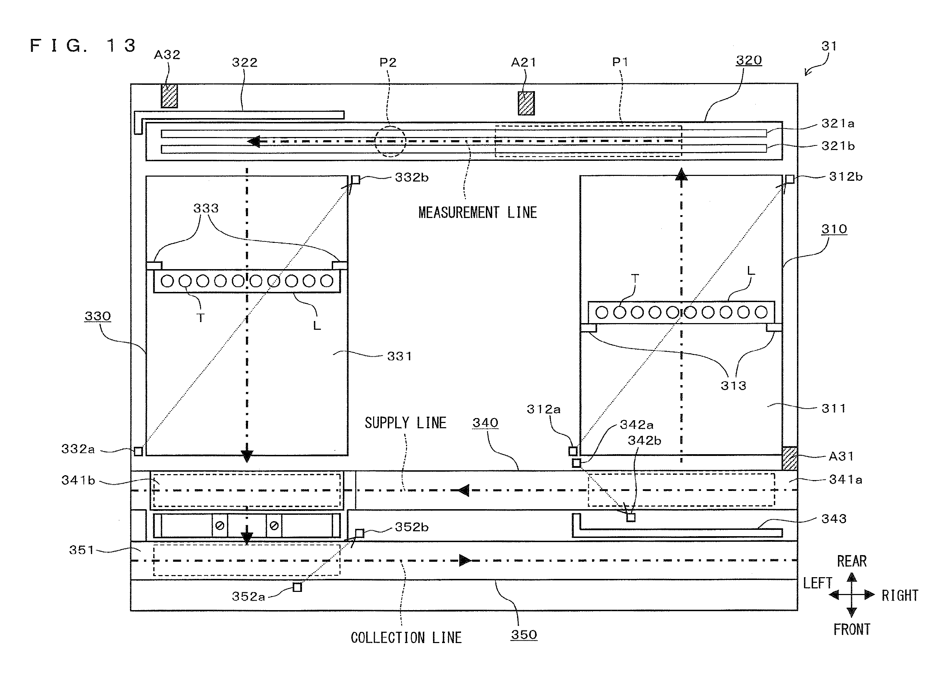

FIG. 13 shows a configuration of a transporting unit according to Embodiment 2 viewed from above;

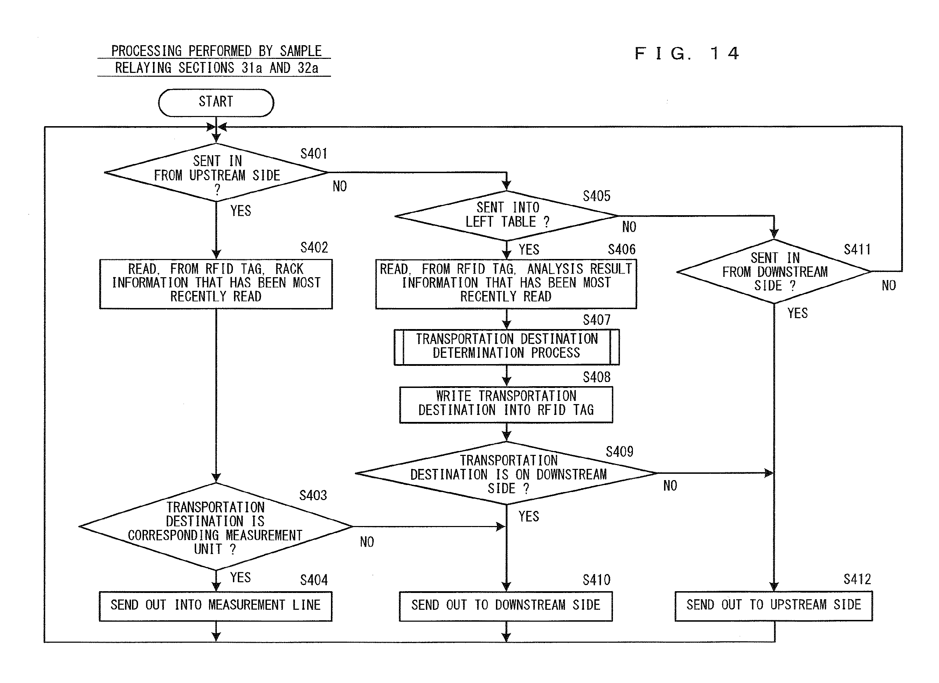

FIG. 14 is a flow chart showing processing performed by a transporting unit according to Embodiment 2;

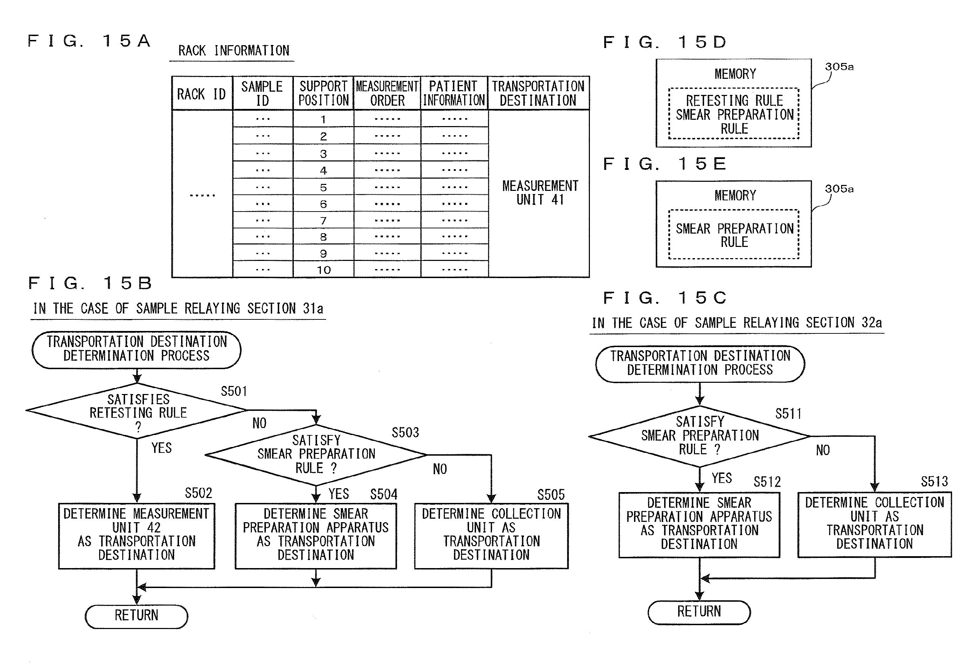

FIG. 15A is a schematic diagram showing a configuration of rack information according to Embodiment 2, each of FIG. 15B and FIG. 15C is a flow chart showing a transportation destination determination process performed by a sample relaying section, and each of FIG. 15D and FIG. 15E is a schematic diagram showing information in a memory of a sample relaying section;

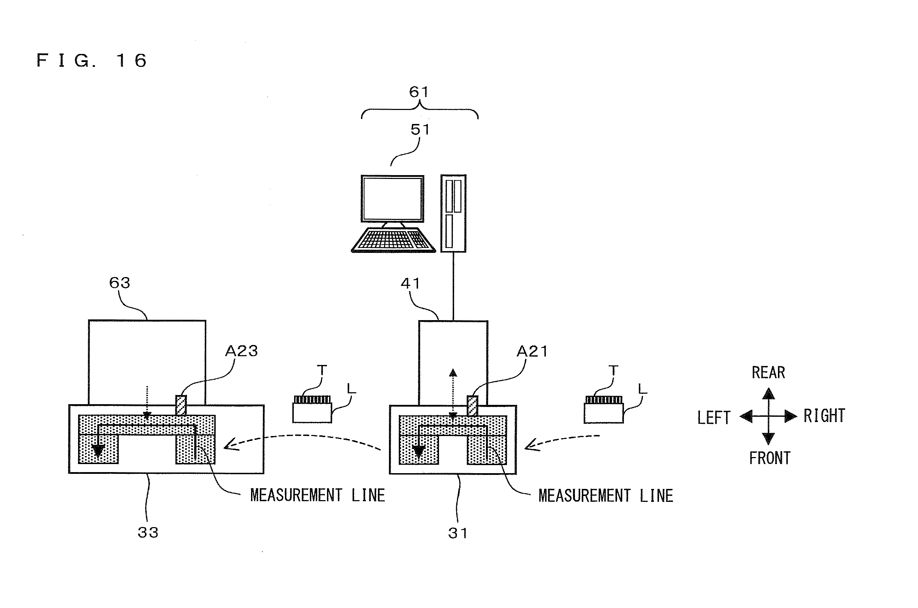

FIG. 16 is a schematic diagram showing configurations of a blood cell analyzer and a smear preparation apparatus according to Embodiment 3 viewed from above; and

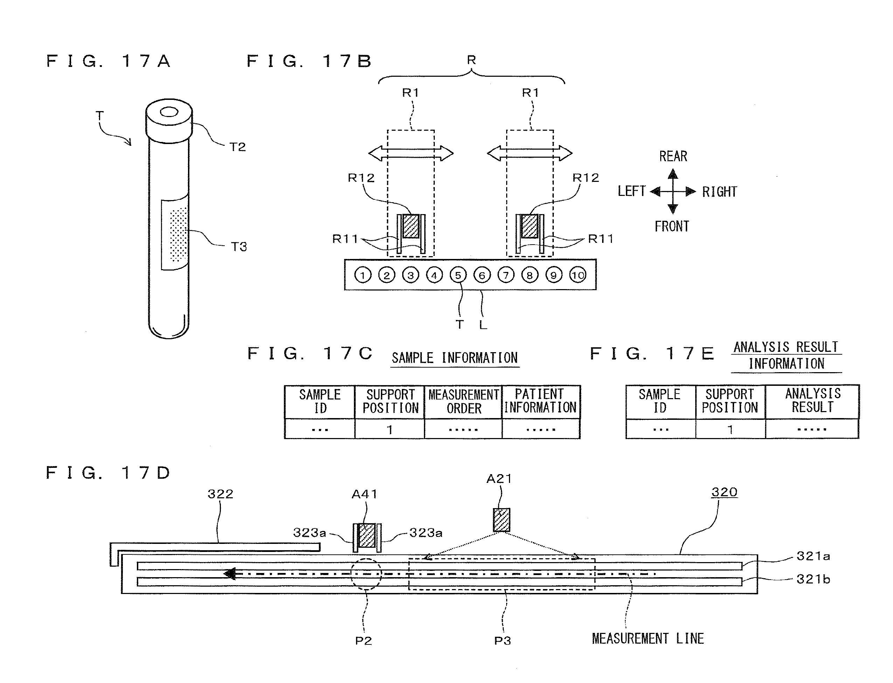

FIG. 17A shows a configuration of a sample container according to Embodiment 4, FIG. 17B is a schematic diagram showing a configuration of an antenna unit according to Embodiment 4 viewed from above, FIG. 17C is a schematic diagram showing a configuration of sample information according to Embodiment 4, FIG. 17D shows a configuration of and around a rack transporter according to Embodiment 4, and FIG. 17E is a schematic diagram showing analysis result information according to Embodiment 4.

### DETAILED DESCRIPTION OF THE EMBODIMENT

Hereinafter, embodiments of the present invention will be described with reference to the drawings.

### <Embodiment 1>

The present embodiment is realized by applying the present invention to a sample processing system for performing tests and analyses of blood. A sample processing system of the present embodiment includes one measurement unit for performing first tests, one measurement unit for performing retests, and one smear preparation apparatus. Blood analyses are performed in parallel in the two measurement units. When preparation of a smear is necessary based on the analysis results, the smear preparation apparatus prepares a smear. In Embodiment 1, a sample rack L sent out downstream from a preprocessing unit 22 is transported on measurement lines of transporting units 31 to 33, in this order, regardless of whether measurement (retest) by a measurement unit 42 and smear preparation by a smear preparation apparatus 63 are necessary.

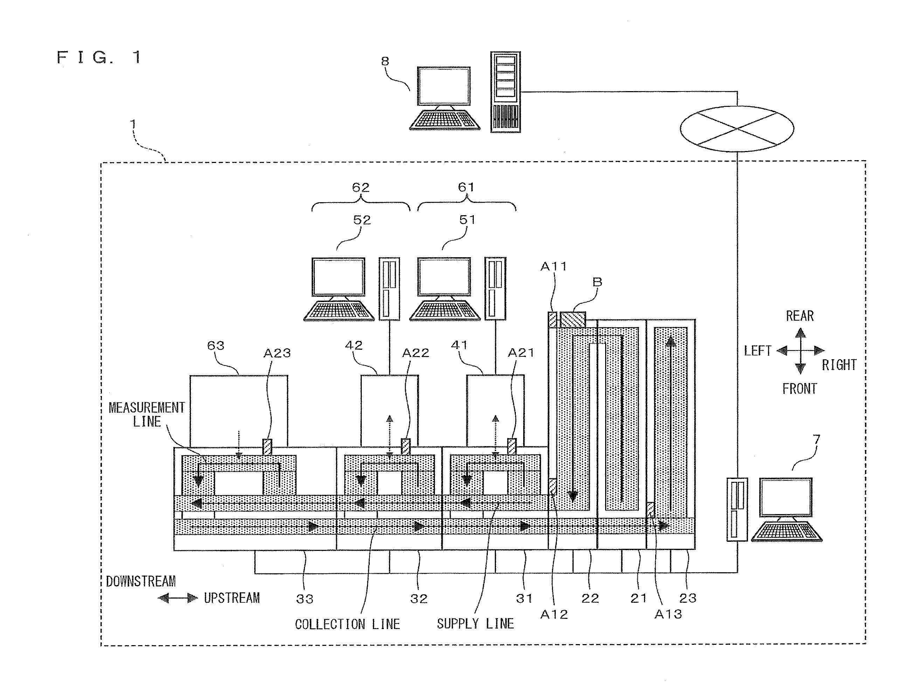

FIG. 1 is a schematic diagram showing a configuration of a sample processing system 1 viewed from above.

The sample processing system 1 according to the present embodiment includes a loading unit 21, the preprocessing unit 22, a collection unit 23, the transporting units 31 to 33, blood cell analyzers 61 and 62, the smear preparation apparatus 63, and a transportation controller 7. The blood cell analyzer 61 includes an information processing unit 51, a measurement unit 41, and a sample supplying section 31b (see FIG. 6) which is a part of the transporting unit 31. The blood cell analyzer 62 includes an information processing unit 52, the measurement unit 42, a sample supplying section 32b (see FIG. 6) which is a part of the transporting unit 32. The sample processing system 1 of the present embodiment is communicably connected to a host computer 8 via a communication network.

The loading unit 21, the preprocessing unit 22, the collection unit 23, and the transporting units 31 to 33 are arranged next to their adjacent units in the left-right direction such that a sample rack L can be transported therebetween. That is, each of these units is configured to be able to receive a sample rack L sent out from a unit located to its upstream side (right) and send out the sample rack L to its downstream side (left) or its upstream side (right). Further, each of these units is configured such that a plurality of sample racks L, each being capable of holding 10 sample containers T, can be placed on the unit.

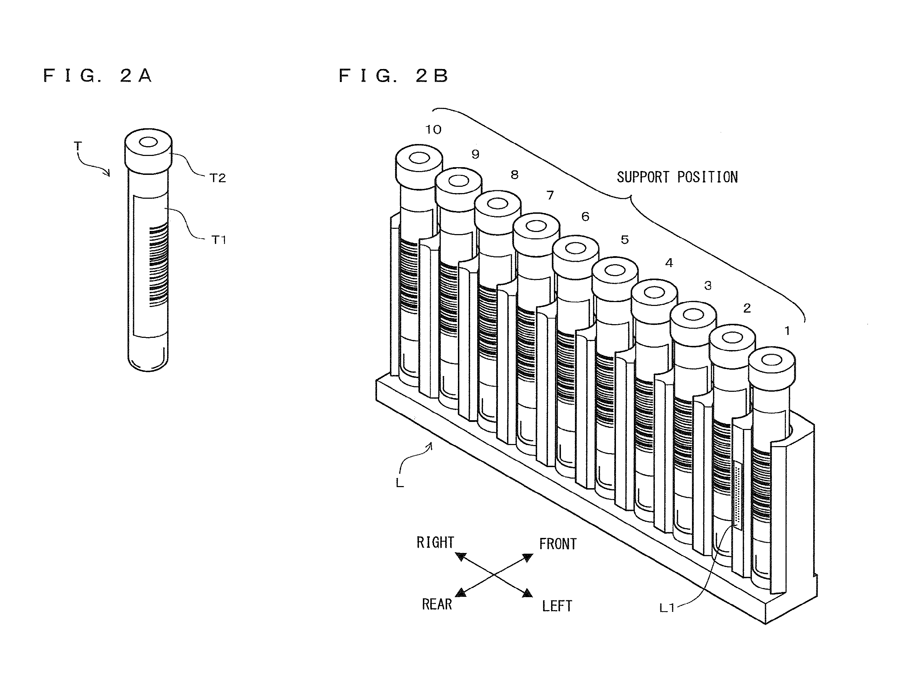

FIG. 2A and FIG. 2B show configurations of a sample container T and a sample rack L, respectively. FIG. 2A is a perspective view showing an external view of a sample container T, and FIG. 2B is a perspective view showing an external view of a sample rack L holding 10 sample containers T. FIG. 2B also shows the orientation (the front, rear, left, and right directions shown in FIG. 1) of a sample rack L when it is placed on the loading unit 21.

With reference to FIG. 2A, each sample container T is a tubular container made of translucent glass or synthetic resin, and its upper end is open. A bar code label T1 is attached to a lateral side of the sample container T. A bar code including a sample ID is printed on the bar code label T1. The sample container T contains a whole blood sample collected from a patient (subject), and the opening at the upper end thereof is sealed with a rubber cap T2.

With reference to FIG. 2B, an RFID (radio frequency identification) tag L1 is attached to a rear lateral side of each sample rack L. In the RFID tag L1, a rack ID is stored as an initial state. In addition, holders capable of vertically holding 10 sample containers T are formed in the sample rack L. Hereinafter, the positions of the holders are referred to as support positions 1 to 10, respectively, from downstream to upstream in the transporting direction, in the ascending order.

With reference back to FIG. 1, the loading unit 21 accommodates a sample rack L loaded by a user, and sends out the sample rack L accommodated thereon to the preprocessing unit 22. When starting measurement of samples, the user sets sample containers T each containing a sample, in a sample rack L, and places this sample rack L on the loading unit 21. Then, this sample rack L is sequentially transported to units on the downstream side (left), and processing is performed.

The preprocessing unit 22 reads out, by means of an antenna A11, a rack ID from the RFID tag L1 of the sample rack L sent out from the loading unit 21. Further, the preprocessing unit 22 reads a sample ID of each sample container T and detects the support position at which each sample container T is held in the sample rack L, by means of a bar code unit B. Then, the preprocessing unit 22 transmits such information to the transportation controller 7 and receives measurement orders and patient information from the transportation controller 7.

Further, the preprocessing unit 22 writes, by means of an antenna A12, the information obtained by the antenna A11 and the bar code unit B and the measurement orders and the patient information received from the transportation controller 7, into the RFID tag L1 of the sample rack L; and then sends out this sample rack L to the transporting unit 31. The information written into the RFID tag L1 will be described later with reference to FIG. 4C.

The measurement unit 41, the measurement unit 42, and the smear preparation apparatus 63 are arranged to the rear of the transporting units 31 to 33, respectively. Each of the transporting units 31 to 33 is provided with two transport lines to be respectively used in a case where a sample rack L is transported to a unit or apparatus arranged to the rear side thereof, and in a case where a sample rack L is not transported to the unit or apparatus arranged to the rear side thereof. In the case where processing is performed in the rear unit or apparatus, the sample rack L is transported along a "measurement line" indicated by an arrow shown on the rear side. In the case where processing is not performed in the rear unit or apparatus but is performed on its downstream side, the sample rack L is transported along a "supply line" indicated by a left arrow shown in the middle so as to skip the rear unit or apparatus.

Further, each of the transporting units 31 to 33 is provided with a transport line for transporting a sample rack L to the collection unit 23. A sample rack L for which processing on the downstream side (left) is no longer necessary is transported along a "collection line" indicated by a right arrow shown on the front, to be collected into the collection unit 23.

When a sample rack L sent out from an upstream unit is transported along the measurement line of the transporting units 31, 32, or 33, a corresponding one of antennas A21 to A23 arranged at a predetermined position on each measurement line reads information from the RFID tag L1 of the sample rack L. The information read from the RFID tag L1 will be described later with reference to FIG. 4C and FIG. 4D.

Each of the measurement units 41 and 42 takes out a sample container T from the sample rack L, at a predetermined position (dotted arrow in FIG. 1) on its measurement line, and measures the sample contained in this sample container T. Specifically, each of the measurement units 41 and 42 moves rearward the sample container T taken out from the sample rack L to take it therein, aspirates the sample from the sample container T, and measures the aspirated sample. Upon completion of the measurement, each of the measurement units 41 and 42 returns the sample container T to its original holder in the sample rack L.

In the present embodiment, the measurement unit 41 is used for first tests (first measurement) and the measurement unit 42 is used for retests (second measurement). The measurement unit 41 can perform measurement for CBC items and DIFF items, and the measurement unit 42 can perform measurement for CBC items, DIFF items, and RET items. The CBC items include WBC (white blood cell), RBC (red blood cell), PLT (platelet), HGB (hemoglobin), HCT (hematocrit), and the like. The DIFF items include MONO (monocyte), EO (eosinophil), BASO (basophil), NEUT (neutrophil), LYMPH (lymphocyte), and the like. The RET items include RET (reticulocyte) and the like.

The information processing units 51 and 52 control operations of the sample supplying section 31b (see FIG. 6) which is a part of the transporting unit 31, and the sample supplying section 32b (see FIG. 6) which is a part of the transporting unit 32, respectively. Further, the information processing units 51 and 52 control operations of the measurement units 41 and 42 based on information read by the antennas A21 and A22, respectively. Further, the information processing units 51 and 52 receive and analyze measurement data of samples from the measurement units 41 and 42, respectively, and generate analysis results including analysis values such as a white blood cell count, a red blood cell count, a platelet count, a hemoglobin concentration, a hematocrit, a monocyte count, and a reticulocyte count.

When measurements by the measurement unit 41 (42) have been completed for all the sample containers T held in the sample rack L, analysis results are written in the RFID tag L1 of this sample rack L by the antenna A21 (A22).

The smear preparation apparatus 63 controls operations of the transporting unit 33. Based on information read by the antenna A23, the smear preparation apparatus 63 aspirates a sample from a sample container T held in the sample rack L at a predetermined position (dotted arrow in FIG. 1) on the measurement line of the transporting unit 33, and prepares a smear of the aspirated sample.

The collection unit 23 reads analysis results, by means of an antenna A13, from an RFID tag L1 of a sample rack L transported on the collection line from the downstream side. Further, the collection unit 23 transmits the read analysis results to the transportation controller 7 and stores the sample rack L from which the analysis results have been read.

The transportation controller 7 controls transporting operations of the loading unit 21, the preprocessing unit 22, the collection unit 23, a sample relaying section 31a (see FIG. 6) which is a part of the transporting unit 31, a sample relaying section 32a (see FIG. 6) which is a part of the transporting unit 32, and the transporting unit 33. Upon receiving information obtained by the antenna A11 and the bar code unit B from the preprocessing unit 22, the transportation controller 7 transmits the rack ID and the sample IDs to the host computer 8, and transmits to the preprocessing unit 22 information such as measurement orders and the like received from the host computer 8. Further, the transportation controller 7 transmits to the host computer 8 analysis results received from the collection unit 23.

The host computer 8 have stored therein, associated with a sample ID, test information and the like that includes: information of a patient from whom a sample corresponding to the sample ID has been collected; a measurement order including items for which measurements should be performed on this sample; past analysis results and illness information of this patient, and the like. It should be noted that a user can designate, in the host computer 8, a measurement item for which measurement should be performed, by using an input device such as a mouse.

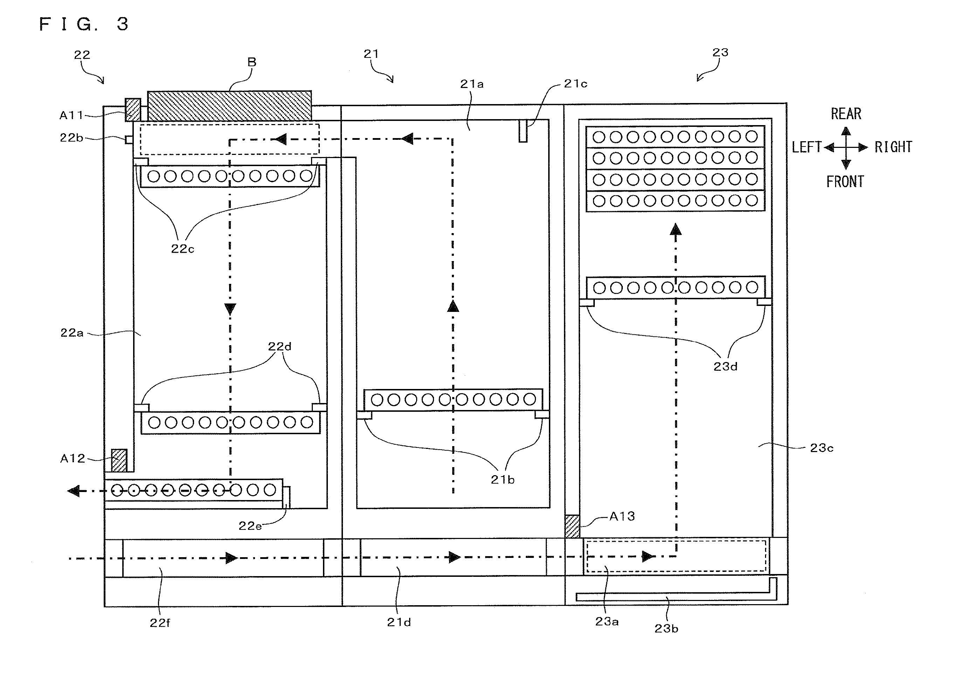

FIG. 3 shows configurations of the loading unit 21, the preprocessing unit 22, and the collection unit 23 viewed from above.

When a sample rack L holding sample containers T is placed on a transport path 21a of the loading unit 21, the sample rack L is sent to a rear position of the transport path 21a, by a front face of the sample rack L being pushed by a rack send-in mechanism 21b. The sample rack L located at the rear position of the transport path 21a is sent out to a rear position of a transport path 22a of the preprocessing unit 22, by a right-side face of the sample rack L being pushed by a rack send-out mechanism 21 c.

A reflective sensor 22b is provided near the rear position of the transport path 22a of the preprocessing unit 22. When the sensor 22b has detected that the sample rack L sent out from the loading unit 21 has been located at the rear position of the transport path 22a, the antenna A11 reads the rack ID from the RFID tag L1 of the sample rack L, and the bar code unit B reads the sample IDs associated with their respective support positions of the sample rack L.

FIG. 4A is a schematic diagram showing a configuration of the bar code unit B viewed from above.

The bar code unit B includes two moving parts B1 provided in parallel to each other in the left-right direction. The two moving parts B1 are configured to be able to move in the left-right direction. Each moving part B1 includes two rollers B11, a roller B12, and a bar code reader B13. Each of the two rollers B11 is configured to be rotatable about an axis in the up-down direction on the moving part B1, and is configured to be able to be driven in the front-rear direction on the moving part B1. The roller B12 is configured to be rotatable about an axis in the up-down direction on the moving part B1. The bar code reader B13 is fixed to the moving part B1 and reads a sample ID from a bar code label T1 of a sample container T located in front of it.

When the bar code reader B13 is located in front (to the rear side) of a support position of a sample rack L, the two rollers B11 are moved forward so as to come into contact with a lateral surface of a corresponding sample container T. At this time, as shown in FIG. 4B, a front portion of the lateral surface of the sample container T is in contact with the roller B12. When the rollers B11 are moved forward and the rollers B11 can be moved further forward than a predetermined moving width, it is determined that no sample container T is held at this support position. On the other hand, when the rollers B11 cannot be moved further forward than the predetermined moving width by contacting a sample container T, it is determined that a sample container T is being held at this support position.

When it has been determined that a sample container T is being held at the support position in front of the bar code reader B13, the roller B12 is rotated, the sample container T is accordingly rotated about an axis in the up-down direction, and the sample ID is accordingly read from the bar code label T1 by the bar code reader B13 during the rotation of the sample container T. It should be noted that, the bar code reader B13 of the left moving part B1 reads sample IDs of sample containers T held at the support positions 1 to 5, and the bar code reader B13 of the right moving part B1 reads sample IDs of sample containers T held at the support positions 6 to 10.

With reference back to FIG. 3, the rack ID read by the antenna A11 and the sample IDs and the support positions of the sample containers T obtained by the bar code unit B are transmitted to the transportation controller 7. The transportation controller 7 transmits the information to the host computer 8 and receives measurement orders and patient information from the host computer 8. Based on the information obtained by the antenna A11 and the bar code unit B and the measurement orders and patient information received from the host computer 8, the transportation controller 7 generates "rack information" as shown in FIG. 4C, and stores this rack information in a hard disk 703 (see FIG. 7).

The sample rack L from which the above information has been obtained by the antenna A11 and the bar code unit B is sent to a front position of the transport path 22a by a rear face of the sample rack L being pushed by rack send-in mechanisms 22c and 22d. The sample rack L located at the front position of the transport path 22a is moved leftward by a right-side face of the sample rack L being pushed by a rack send-out mechanism 22e.

At this time, the antenna A12 reads the rack ID from the RFID tag L1 of the sample rack L. The preprocessing unit 22 transmits the rack ID read by the antenna A12, to the transportation controller 7. The transportation controller 7 transmits, to the preprocessing unit 22, rack information corresponding to the rack ID received from the preprocessing unit 22 among pieces of rack information stored in the hard disk 703. Then, the preprocessing unit 22 writes the rack information received from the transportation controller 7 into the RFID tag L1 of the sample rack L, by using the antenna A12. Then, this sample rack L is pushed further leftward by the rack send-out mechanism 22e, to be sent out into the transporting unit 31.

Meanwhile, a sample rack L that has been sent out from the measurement unit 41 or 42, or the smear preparation apparatus 63 into the preprocessing unit 22 along the collection line is transported by a belt 22f of the preprocessing unit 22, a belt 21d of the loading unit 21, and a belt 23a of the collection unit 23, to be located at a front position of the collection unit 23 (which corresponds to the belt 23a).

When the sample rack L is located at the front position of the collection unit 23, the antenna A13 reads "analysis result information" written in the RFID tag L1 of the sample rack L. As shown in FIG. 4D, "analysis result information" which includes analysis results of samples measured in the measurement units 41 and 42 is stored in the RFID tag L1. Analysis result information is individually stored for each sample. When measurements by the measurement units 41 and 42 are completed, each of the antennas A21 and A22 writes corresponding analysis result information into the RFID tag L1. In the initial state, nothing is stored as analysis result information. The collection unit 23 transmits the analysis results read by the antenna A13, to the transportation controller 7. The transportation controller 7 transmits the analysis results received from the collection unit 23, to the host computer 8.

The sample rack L for which the reading by the antenna A13 has been completed is pushed out by a rack push-out mechanism 23b, from the front position of the collection unit 23 onto a transport path 23c. The sample rack L pushed out onto the transport path 23c is sent to a rear position of the transport path 23c by its front face being pushed by a rack send-in mechanism 23d. In this manner, each sample rack L holding sample containers T for which measurements have been completed is sequentially collected in the rear of the transport path 23c.

FIG. 5 shows a configuration of the transporting unit 31 viewed from above.

The transporting unit 31 includes a right table 310, a rack transporter 320, a left table 330, and rack transporters 340 and 350. The right table 310, the rack transporter 320, and the left table 330 form the measurement line shown in FIG. 1. The rack transporter 340 forms the supply line shown in FIG. 1. The rack transporter 350 forms the collection line shown in FIG. 1.

When measurement for a sample rack L sent out from the preprocessing unit 22 on the upstream side is not performed in the measurement unit 41, the sample rack L is linearly sent along the supply line from the right end to the left end of the rack transporter 340, by belts 341a and 341b of the rack transporter 340. Then, this sample rack L is sent out into the transporting unit 32 on the downstream side by the belt 341b. It should be noted that, in the present embodiment, as described below, all sample racks L are transported along the measurement lines of the transporting units 31 to 33, and thus, they are not transported along the supply line.

Next, when measurement for the sample rack L sent out from the preprocessing unit 22 on the upstream side is performed in the measurement unit 41, this sample rack L is located at a right end position of the rack transporter 340. When the sample rack L has been detected by transmissive sensors 342a and 342b provided near the right end position of the rack transporter 340, the sample rack L is pushed out to a front position of a transport path 311 of the right table 310, by its front face being pushed by a rack push-out mechanism 343.

When the sample rack L on the transport path 311 has been detected by transmissive sensors 312a and 312b provided on the right table 310, the sample rack L is sent rearward by its front face being pushed by a rack send-in mechanism 313. In this manner, the sample rack L is sent to a right end position of the rack transporter 320.

Belts 321a and 321b of the rack transporter 320 can be independently driven in the left-right direction by different stepping motors (not shown). Further, each of the belts 321a and 321b is provided with projections (not shown) which engage the bottom face of each sample rack L. The sample rack L is transported in the left-right direction along the rack transporter 320, by being held by the projections of the belt 321a or the belt 321b.

The sample rack L transported leftward by the belt 321a or the belt 321b from the right end position of the rack transporter 320 is located near a position P1. When the sample rack L is located at the position P1, the antenna A21 reads rack information shown in FIG. 4C from the RFID tag L1 of this sample rack L. The read rack information is transmitted to the information processing unit 51.

When the sample rack L is moved leftward further from the position P1 and a sample container T is located at a position P2, a hand part (not shown) of the measurement unit 41 takes out the sample container T located at the position P2 from the sample rack L and takes it inside the measurement unit 41. The information processing unit 51 causes the measurement unit 41 to operate and measure the sample that has been taken in. At this time, the information processing unit 51 determines measurement items for the sample based on the measurement order included in the rack information read by the antenna A21. When the measurement in the measurement unit 41 has been completed, the sample container T is returned to its original support position in the sample rack L.

In this manner, when measurements of all the samples held in the sample rack L have been completed, the sample rack L is moved to the position P1 again by the belt 321a or 321b. Then, the antenna A21 writes, into the RFID tag L1 of the sample rack L, an analysis result of each sample measured by the measurement unit 41 as analysis result information as shown in FIG. 4D.

When the writing by the antenna A21 has been completed, the sample rack L is sent to a left end position of the rack transporter 320. Then, the sample rack L is pushed out to a rear position of a transport path 331 of the left table 330, by means of a rack push-out mechanism 322. When the sample rack L on the transport path 331 has been detected by transmissive sensors 332a and 332b provided on the left table 330, the sample rack L is sent forward by its rear face being pushed by a rack send-in mechanism 333, and is located at a left end position of the rack transporter 340 or a left end position of the rack transporter 350.

In the case where the sample rack L is sent out to the transporting unit 32 on the downstream side, the sample rack L is sent out into the transporting unit 32 on the downstream side by the belt 341b of the rack transporter 340, from the left end position of the rack transporter 340.

On the other hand, in the case where the sample rack L is sent out into the preprocessing unit 22 on the upstream side, the sample rack L detected by transmissive sensors 352a and 352b provided near the left end position of the rack transporter 350 is linearly sent along the collection line from the left end to the right end of the rack transporter 350, by means of a belt 351 of the rack transporter 350, and then sent out into the preprocessing unit 22 on the upstream side. Then, the sample rack L transported rightward along the collection line is stored in the collection unit 23 in the end.

Each of the transporting units 32 and 33 also has a configuration similar to that of the transporting unit 31 as described below.

In the transporting unit 32, when a sample rack L is located at the position P1 of the transporting unit 32, the antenna A22 reads rack information and analysis result information from the RFID tag L1 of the sample rack L. The read rack information and analysis result information are transmitted to the information processing unit 52. Further, the antenna A22 writes, into the RFID tag L1, an analysis result of each sample measured by the measurement unit 42 as analysis result information.

In the transporting unit 33, when a sample rack L is located at the position P1 of the transporting unit 33, the antenna A23 reads rack information and analysis result information from the RFID tag L1 of the sample rack L. The read rack information and analysis result information are transmitted to the smear preparation apparatus 63.

FIG. 6 shows connection relationship between units and apparatuses of the sample processing system 1.

The transporting unit 31 is shown, divided into the sample relaying section 31a and the sample supplying section 31b. The transporting unit 32 is shown, divided into the sample relaying section 32a and the sample supplying section 32b. Specifically, each of the sample relaying sections 31a and 32a corresponds to a part including the left table 330 and the rack transporters 340 and 350 shown in FIG. 5, and receives a sample rack L from one of two adjacent units and transports it to the other transporting unit. Each of the sample supplying sections 31b and 32b corresponds to a part including the right table 310 and the rack transporter 320 shown in FIG. 5, and transports a sample rack L to the positions P1 and P2 for measurement by its corresponding measurement units 41 or 42. It should be noted that the transporting unit 33 includes all components shown in FIG. 5.

The loading unit 21, the preprocessing unit 22, the collection unit 23, the sample relaying sections 31a and 32a, the transporting unit 33, and the transportation controller 7 are communicably connected to a concentrator 11. The information processing unit 51 is communicably connected to the sample relaying section 31a, the sample supplying section 31b, and the measurement unit 41. The information processing unit 52 is communicably connected to the sample relaying section 32a, the sample supplying section 32b, and the measurement unit 42. The smear preparation apparatus 63 is communicably connected to the transporting unit 33. The transportation controller 7 is communicably connected to the host computer 8 via a communication network.

FIG. 7 is a schematic diagram showing configurations of the preprocessing unit 22, the collection unit 23, and the transportation controller 7.

The preprocessing unit 22 includes a controller 221, a communication section 222, the bar code unit B, the antennas A11 and A12, a driving section 223, and a sensor section 224. The controller 221 includes a memory 221a.

The controller 221 controls components included in the preprocessing unit 22 by executing computer programs stored in the memory 221a. The communication section 222 performs data communication with the concentrator 11.

Information obtained by the bar code unit B and the antennas A11 and A12 is outputted to the controller 221. The controller 221 transmits the information to the transportation controller 7 via the communication section 222. Further, the controller 221 stores generated rack information (see FIG. 4C) in the memory 221a. The driving section 223 includes mechanisms for transporting each sample rack L on the preprocessing unit 22. The sensor section 224 includes sensors for detecting a sample rack L on the preprocessing unit 22.

The collection unit 23 includes a controller 231, a communication section 232, the antenna A13, a driving section 233, and a sensor section 234. The controller 231 includes the memory 231a. The collection unit 23 has a configuration equivalent to that of the preprocessing unit 22 from which the bar code unit B, the antennas A11 and A12 are eliminated and to which the antenna A13 is add. Analysis result information read by the antenna A13 is outputted to the controller 231. The controller 231 transmits the analysis result information to the transportation controller 7 via the communication section 232.

The transportation controller 7 includes a controller 701, a communication section 702, the hard disk 703, and a display input unit 704. The controller 701 includes a memory 701a.

The controller 701 controls the loading unit 21, the preprocessing unit 22, the collection unit 23, the sample relaying sections 31a and 32a, and the transporting unit 33, by executing computer programs stored in the memory 701a or in the hard disk 703. The communication section 702 performs data communication with the concentrator 11 and performs data communication with the host computer 8 via a communication network.

Computer programs for controlling target units are stored in the hard disk 703. The display input unit 704 includes a display and an input device.

FIG. 8 is a schematic diagram showing configurations of the transporting unit 31 (the sample relaying section 31a and the sample supplying section 31b), the measurement unit 41, and the information processing unit 51. Also, the transporting unit 32, the measurement unit 42, and the information processing unit 51 have configurations similar to those shown in FIG. 8, respectively.

The sample relaying section 31a includes a controller 301a, a communication section 302a, a driving section 303a, and a sensor section 304a. The controller 301a includes a memory 305a. The controller 301a controls components included in the sample relaying section 31a, by executing computer programs stored in the memory 305a. The communication section 302a performs data communication with the information processing unit 51 and the concentrator 11. Detection signals from the sensor section 304a are outputted to the controller 301a. The driving section 303a and the sensor section 304a respectively include mechanisms for transporting and detecting sample racks L that are on the left table 330, and the rack transporters 340 and 350 shown in FIG. 5.

The sample supplying section 31b includes a communication section 301b, the antenna A21, a driving section 302b, and a sensor section 303b. Each of the components included in the sample supplying section 31b is controlled by the information processing unit 51 via the communication section 301b. The communication section 301b performs data communication with the information processing unit 51. Information read by the antenna A21 and detection signals from the sensor section 303b are outputted to the information processing unit 51 via the communication section 301b. The driving section 302b and the sensor section 303b respectively include mechanisms for transporting and detecting sample racks L that are on the right table 310 and the rack transporter 320 shown in FIG. 5.

The measurement unit 41 includes a communication section 411, a driving section 412, a sensor section 413, a specimen preparation section 414, and a detector 415. Each of the components included in the measurement unit 41 is controlled by the information processing unit 51 via the communication section 411. The communication section 411 performs data communication with the information processing unit 51. The driving section 412 includes mechanisms for measuring a sample. The sensor section 413 includes sensors and the like for detecting a position of a sample container T in the measurement unit 41. The specimen preparation section 414 mixes and agitates a reagent and a sample aspirated in the measurement unit 41 to prepare a specimen for measurement. The detector 415 measures the specimen prepared by the specimen preparation section 414. Measurement data of the sample obtained through the measurement is transmitted to the information processing unit 51 via the communication section 411, and is subjected to analysis in the information processing unit 51.

It should be noted that measurement of white blood cells, reticulocytes, and the like is performed by detecting scattered light and fluorescence through flow cytometry using a semiconductor laser. Further, measurement of red blood cells and platelets is performed by detecting changes in impedance through a sheath flow DC detection method. Further, measurement of a hemoglobin (HGB) concentration is performed by detecting absorbance. Measurement data (digital data) including scattered light intensity, fluorescence intensity, and impedance change of a sample is calculated based on data obtained by the detector 415. The measurement data is analyzed by the information processing unit 51, to generate an analysis result including analysis values such as a red blood cell count, a white blood cell count, and a reticulocyte count.

The information processing unit 51 includes a controller 511, a communication section 512, a hard disk 513, and a display input unit 514. The controller 511 includes a memory 511a. The information processing unit 51 has a configuration similar to that of the transportation controller 7 shown in FIG. 7.

The controller 511 controls components included in the information processing unit 51. The communication section 512 performs data communication with the sample relaying section 31a, the sample supplying section 31b, and the measurement unit 41. Further, the controller 511 analyzes measurement data of a sample received from the measurement unit 41, generates an analysis result, and stores it in the hard disk 513. The display input unit 514 displays an analysis result on a display based on video signals outputted from the controller 511, and receives an input from a user via an input device.

FIG. 9 is a schematic diagram showing configurations of the transporting unit 33 and the smear preparation apparatus 63.

The transporting unit 33 includes a controller 331, a communication section 332, the antenna A23, a driving section 333, and a sensor section 334. The controller 331 includes a memory 331a. The controller 331 controls components included in the transporting unit 33, by executing computer programs stored in the memory 331a. The communication section 332 performs data communication with the smear preparation apparatus 63. The driving section 333 includes mechanisms for driving components included in the transporting unit 33. The sensor section 334 includes sensors in the transporting unit 33, and detection signals from the sensor section 334 are outputted to the controller 331.

The smear preparation apparatus 63 includes a controller 631, a communication section 632, a driving section 633, a sensor section 634, a dispenser 635, a spreader glass driving section 636, and a display input unit 637. The controller 631 includes a memory 631a.

The controller 631 controls components included in the smear preparation apparatus 63, by executing computer programs stored in the memory 631a. The communication section 632 performs data communication with the transporting unit 33. The driving section 633 includes mechanisms for driving components included in the smear preparation apparatus 63. The sensor section 634 includes sensors and the like in the smear preparation apparatus 63, and detection signals from the sensor section 634 are outputted to the controller 631.

The dispenser 635 aspirates a sample form a sample container T at the position P2 on the rack transporter 320 of the transporting unit 33, and dispenses the sample on a slide glass. The spreader glass driving section 636 drives a spreader glass. The controller 631 controls the spreader glass driving section 636, to adjust a moving speed of a spreader glass, an angle of the spreader glass relative to a slide glass, and a time period (fitting time) from immediately after the spreader glass is made in contact with a sample dispensed on the slide glass until the spreader glass starts to move.

The display input unit 637 is a touch panel in which a displaying function and an inputting function are integrated. When the display input unit 637 is operated by a user, a signal indicating the content of the operation is outputted to the controller 631. Further, the controller 631 causes the display input unit 637 to display various types of information.

FIG. 10 is a flow chart showing processing performed by the information processing units 51 and 52. Since the processing performed by the information processing unit 51 is substantially the same as the processing performed by the information processing unit 52, the processing performed by the information processing unit 51 will be described unless otherwise mentioned.

The controller 511 of the information processing unit 51 determines whether a sample rack L sent into the transporting unit 31 has been pushed out onto the right table 310 (i.e., sent into the measurement line) by the rack push-out mechanism 343 (S101). This is determined based on detection signals from the sensors 312a and 312b. The controller 511 causes the processing to wait until a sample rack L has been sent into the measurement line.

When a sample rack L has been sent into the measurement line (S101: YES), the controller 511 causes the sample rack L to be transported to the position P1, and causes the antenna A21 to read rack information and analysis result information (see FIG. 4C and FIG. 4D) from the RFID tag L1 of the sample rack L (S102).

Next, based on the information read in S102, the controller 511 sequentially determines a measurement content for each sample on the sample rack L as described below.

The controller 511 refers to the analysis result information read in S102, and determines whether a sample on the sample rack L has been subjected to a first test (S103). Specifically, with respect to a sample ID corresponding to the sample, it is determined whether an analysis result exists in the analysis result information. When the sample has not been subjected to a first test (S103: NO), the controller 511 determines first a test item based on a measurement order of this sample in the rack information (S104).

It should be noted that, in the processing performed by the information processing unit 51, none of the samples on a sample rack L on the transporting unit 31 have been measured yet. Therefore, no analysis result exists in the analysis result information, and thus, it is always determined as NO in S103. Further, in the processing performed by the information processing unit 52, all of the samples in the transporting unit 32 have already been measured in the measurement unit 41. Therefore, a corresponding analysis result exists in the analysis result information and it is always determined as YES in S201.

When the sample has been subjected to a first test (S103: YES), the controller 511 determines whether retesting of this sample is necessary based on its analysis result in the analysis result information (S105). Specifically, based on a retesting rule stored in the hard disk 513, it is determined whether the analysis result of this sample satisfies the retesting rule. When the analysis result satisfies the retesting rule (S105: YES), the controller 511 determines a retest item for this sample (S203). When the analysis result does not satisfy the retesting rule (S105: NO), the process of S106 is skipped. The controller 511 repeats the processes of S103 to S106 until the processes of S103 to S106 are performed for all of the samples on the sample rack L (S107).

Next, based on the measurement content determined through S103 to S106, the controller 511 sequentially performs a measurement operation for each sample on this sample rack L as described below.

Based on the measurement content determined through S103 to S106, the controller 511 determines whether measurement is necessary for a sample on the sample rack L (S108). Specifically, when S104 and S106 have been performed, it is determined as YES in S108; and when it has been determined as NO in S105, it is determined as NO in S108.

When measurement of the sample is necessary (S108: YES), the controller 511 causes the measurement unit 41 to perform a measurement operation on this sample (S109). When measurement of the sample is not necessary (S108: NO), the controller 511 skips the process of S109. The controller 511 repeats the processes of S108 and S109 until the processes of S108 and S109 are performed for all of the samples on the sample rack L (S110).

Next, when any of the samples on the sample rack L has been measured in the measurement unit 41 (S111: YES), the controller 511 causes the sample rack L to be transported to the position P1, and causes an analysis result of the sample that has been measured to be written in the RFID tag L1, to update the analysis result information stored in the RFID tag L1 (S112). When none of the samples on the sample rack L has been measured in the measurement unit 41 (S111: NO), the controller 511 skips the process of S112.

Then, the controller 511 causes the rack push-out mechanism 322 to push out this sample rack L onto the left table 330, and returns the processing to S101. In this manner, processing of one sample rack L is completed.

FIG. 11A is a flow chart showing processing performed by the transporting unit 33.

The controller 331 of the transporting unit 33 determines, as in S101 shown in FIG. 10, whether a sample rack L sent into the transporting unit 33 has been sent into the measurement line (S201). When a sample rack L has been sent into the measurement line (S201: YES), the controller 331 causes the antenna A23 to read rack information and analysis result information from the RFID tag L1 of the sample rack L, as in S102 shown in FIG. 10. Then, the controller 331 transmits the rack information and the analysis result information read in S202 to the smear preparation apparatus 63 (S203). Other processes performed by the transporting unit 33 will be described later with reference to FIG. 12.

FIG. 11B is a flow chart showing processing performed by the smear preparation apparatus 63.

When the controller 631 of the smear preparation apparatus 63 has received the rack information and the analysis result information transmitted from the transporting unit 33 in S203 shown in FIG. 11A (S211: YES), the controller 631 sequentially determines a smear preparation content for each sample on the sample rack L as described below.

The controller 631 refers to the analysis result information received in S211, and determines whether it is necessary to prepare a smear for a sample on the sample rack L (S212). Specifically, it is determined whether it is necessary to prepare a smear, based on whether the analysis result included in the analysis result information that corresponds to the sample on the sample rack L satisfies a smear preparation rule stored in the hard disk 513. When measurement (retesting) has been performed in the measurement unit 42, it is determined whether it is necessary to prepare a smear based on the analysis result of the sample measured by the measurement unit 42.

When it is necessary to prepare a smear (S212: YES), the controller 631 determines a smear preparation condition based on HCT (hematocrit) included in the analysis result of this sample (S213). Specifically, smear preparation conditions corresponding to HCT (hematocrit) values are stored in the memory 631a, and based on a corresponding smear preparation condition, determined are: a moving speed of a spreader glass for spreading a sample dispensed on a slide glass; an angle of the spreader glass relative to the slide glass; and a time period (fitting time) from immediately after the spreader glass is made in contact with the sample dispensed on the slide glass until the spreader glass starts to move. The controller 631 repeats the processes of S212 and S213 until the processes of S212 and S213 are performed for all of the samples on the sample rack L (S214).

Next, based on the smear preparation content determined through S212 and S213, the controller 631 sequentially performs a smear preparation operation for each sample on the sample rack L as described below.

Based on the smear preparation content determined through S212 and S213, the controller 631 determines whether it is necessary to prepare a smear for a sample on the sample rack L (S215). Specifically, when it has been determined as YES in S212, it is determined as YES in S215; and when it is determined as NO in S212, it is determined as NO in S215.

When it is necessary to prepare a smear (S215: YES), the controller 631 performs a preparation operation of a smear of this sample (S216). When it is not necessary to prepare a smear (S215: NO), the controller 631 skips the process of S216. The controller 631 repeats the processes of S215 and S216 until the processes of S215 and S216 are performed for all of the samples on the sample rack L (S217).

Then, the controller 631 causes the rack push-out mechanism 322 to push out this sample rack L onto the left table 330 (S218), and returns the processing to S211. In this manner, processing of one sample rack L is completed.

FIG. 12 is a flow chart showing processing performed by the sample relaying section 31a of the transporting unit 31, the sample relaying section 32a of the transporting unit 32, and the transporting unit 33. It should be noted that substantially the same processing as that shown in FIG. 12 is performed by the sample relaying sections 31a and 32a, and the transporting unit 33, and thus, only the processing performed by the sample relaying section 31a will be described below.

The controller 301a of the sample relaying section 31a causes a sample rack L to be transported to an intended transportation destination as appropriate as described below, depending on the cases where the sample rack L has been sent into the rack transporter 340 from the upstream side, where the sample rack L has been sent into the rack transporter 350 from the downstream side, and where the sample rack L has been sent into the left table 330 from the rack transporter 320.

When the sensors 342a and 342b have detected a sample rack L sent in from the upstream side (S301: YES), the controller 301a causes the rack push-out mechanism 343 to send out this sample rack L onto the right table 310 (measurement line) (S302). The sample rack L sent out onto the right table 310 is transported along the measurement line and is subjected to processing in a corresponding unit or apparatus.

Meanwhile, when the sensors 332a and 332b have detected a sample rack L sent in from the rack transporter 320 (S301: NO, S303: YES), the controller 301a causes this sample rack L to be sent out to the downstream side or the upstream side. When there is a transporting unit on the downstream side (S304: YES), the controller 301a causes this sample rack L to be moved to the rack transporter 340, to be sent out from the rack transporter 340 to the transporting unit 32 on the downstream side (S307). On the other hand, when there is no transporting unit on the downstream side (S304: NO), the controller 301a causes this sample rack L to be moved to the rack transporter 350, to be transported along the collection line, to be sent out to the upstream side (S307).

Meanwhile, when the sensors 352a and 352b have detected a sample rack L sent in from the downstream side (S301: NO, S303: NO, S306: YES), the controller 301a causes this sample rack L to be transported along the collection line, to be sent out to the upstream side (S307).

As described above, according to the present embodiment, an RFID tag L1 is attached to each sample rack L, and rack information regarding samples on the sample rack L is written in this RFID tag L1, in the preprocessing unit 22. Therefore, when a sample rack L is transported to the measurement line of the measurement unit 41 which is for performing first tests, the information processing unit 51 can read the rack information regarding the sample rack L from the RFID tag L1. Accordingly, without inquiring of another apparatus, the information processing unit 51 can determine a first test item for each sample on the sample rack L, based on its corresponding measurement order in the rack information.

Further, according to the present embodiment, when a first test by the measurement unit 41 has been completed, an analysis result of the first test by the measurement unit 41 is written in the RFID tag L1, associated with its corresponding sample ID. Therefore, when a sample rack L has been transported on the measurement line of the measurement unit 42 which is for performing retests, the information processing unit 52 can read the analysis result information regarding each sample on the sample rack L, from the RFID tag L1. Accordingly, without inquiring of another apparatus, the information processing unit 52 can determine whether it is necessary to perform a retest for each sample on the sample rack L and can determine a retest item, based on the analysis result information of its first test performed by the measurement unit 41.

Further, according to the present embodiment, when a retest by the measurement unit 42 has been completed, an analysis result of the retest by the measurement unit 42 is written in the RFID tag L1, associated with its corresponding sample ID. Therefore, when a sample rack L has been transported to the measurement line of the smear preparation apparatus 63, the transporting unit 33 can read, from the RFID tag L1, the analysis result information regarding each sample on the sample rack L, and the smear preparation apparatus 63 can obtain the analysis result information from the transporting unit 33. Accordingly, without inquiring of another apparatus, the smear preparation apparatus 63 can determine whether it is necessary to prepare a smear for each sample on the sample rack L and can determine a smear preparation condition, based on the analysis result information obtained from the measurement units 41 and 42.

As described above, the blood cell analyzers 61 and 62 and the smear preparation apparatus 63 need not communicate with another apparatus (such as the host computer 8) in order to obtain rack information and analysis result information. Therefore, it is possible to suppress communication processing in these apparatuses from being complicated. Further, since these apparatuses need not communicate with another apparatus, it is possible to save time and costs necessary to set computers so as to allow communication therebetween.

Further, according to the present embodiment, when measurement is completed in each of the measurement units 41 and 42, the sample rack L is transported to the position P1, and analysis results are written in the RFID tag L1 of the sample rack L at the position P1. This allows another apparatus to read the analysis results written in the RFID tag L1. Further, the user can save work of moving the sample rack L for which measurement has been completed, to the position P1.

Further, according to the present embodiment, sample IDs obtained by the bar code unit B and support positions of samples associated with the sample IDs are written in the RFID tag L1. Further, analysis results associated with the sample IDs are written in the RFID tag L1. Accordingly, it is possible to read analysis results of the respective samples at one time, simply by reading the RFID tag L1, and it is possible to identify support positions of the respective samples. Accordingly, the measurement unit 42 and the smear preparation apparatus 63 can smoothly advance processing of samples based on their analysis results.

### <Embodiment 2>

In the present embodiment, when measurement (retesting) by the measurement unit 42 and smear preparation by the smear preparation apparatus 63 are not necessary, a transportation of a sample rack L is controlled such that the sample rack L is not loaded on the measurement lines of the transporting units 32 and 33.

FIG. 13 shows a configuration of the transporting unit 31 of the present embodiment viewed from above.

The transporting unit 31 of the present embodiment is configured such that an antenna A31 is provided near the right end position of the rack transporter 340 and an antenna A32 is provided near the left end position of the rack transporter 320 in the transporting unit 31 shown in FIG. 5. The antennas A31 and A32 are controlled by the controller 301a of the sample relaying section 31a (32a), and information read by the antennas A31 and A32 is outputted to the controller 301a of the sample relaying sections 31a (32a). Further, each of the antennas A31 and A32 is always performing a process of reading rack information and analysis result information from an RFID tag L1 located to the front thereof, and the controller 301a stores the read rack information and analysis result information in the memory 305a.

FIG. 14 is a flow chart showing processing performed by the sample relaying section 31a of the transporting unit 31 and by the sample relaying section 32a of the transporting unit 32. It should be noted that substantially the same processing as that shown in FIG. 14 is performed by the sample relaying sections 31a and 32a, and thus, the processing performed by the sample relaying section 31a will be described unless otherwise mentioned. Further, the processing performed by the transporting unit 33 is the same as that shown by the flow chart in FIG. 12, and therefore, description thereof will be omitted.

The controller 301a of the sample relaying section 31a causes a sample rack L to be transported to an intended transportation destination as appropriate as described below, depending on the cases where the sample rack L has been sent into the rack transporter 340 from the upstream side, where the sample rack L has been sent into the left table 330 from the rack transporter 320, and where the sample rack L has been sent into the rack transporter 350 from the downstream side.

It should be noted that as shown in FIG. 15A, a transportation destination is stored associated with rack IDs in the rack information of the present embodiment. The measurement unit 41 is set as an initial value for this transportation destination, when the antenna A12 of the preprocessing unit 22 writes the rack ID into the RFID tag L1.

With reference to FIG. 14, upon detecting a sample rack L sent in from the upstream side (S401: YES), the controller 301a reads rack information that has been most recently read by the antenna A31 from among pieces of rack information stored in the memory 305a (S402). Subsequently, the controller 301a determines whether the transportation destination of the sample rack L whose rack information has been read in S402 is the measurement unit 41 or 42 that corresponds to this transporting unit (S403). For example, in the case of the processing performed by the sample relaying section 31a, it is determined as YES when the transportation destination is the measurement unit 41; and in the case of the processing performed by the sample relaying section 32a, it is determined as YES when the transportation destination is the measurement unit 42.

When the transportation destination is the measurement unit 41 or 42 that corresponds to this transporting unit (S403: YES), the controller 301a causes the sample rack L to be sent out to the measurement line (S404). On the other hand, when the transportation destination is not the measurement unit 41 or 42 that corresponds to this transporting unit (S403: NO), the controller 301a causes the sample rack L to be transported along the supply line to be sent out to the transporting unit 32 on the downstream side (S410).

Further, when the controller 301a has detected a sample rack L sent into the left table 330 from the rack transporter 320 (S401: NO, S405: YES), the controller 301a reads analysis result information that has been most recently read by the antenna A32 from among pieces of analysis result information stored in the memory 305a (S406). Subsequently, the controller 301a performs a "transportation destination determination process" (S407). The transportation destination determination process will be described later with reference to FIG. 15B and FIG. 15C.

Subsequently, the controller 301a causes the antenna A32 to write the transportation destination obtained through the process of S407, into the RFID tag L1 of the sample rack L (S408), and determines whether the written transportation destination is on the downstream side (S409). When the transportation destination is the measurement unit 42 or the smear preparation apparatus 63 on the downstream side (S409: YES), the controller 301a causes this sample rack L to be sent out from the rack transporter 340 to the transporting unit 32 on the downstream side (S410). On the other hand, when the transportation destination is not on the downstream side, that is, the transportation destination is the collection unit 23 (S409: NO), the controller 301a causes the sample rack L to be transported along the collection line to be sent out to the upstream side (S412).

Further, when the controller 301a has detected a sample rack L sent in from the downstream side (S401: NO, S405: NO, S411: YES), the controller 301a causes this sample rack L to be transported along the collection line to be sent out to the upstream side (S412).

FIG. 15B is a flow chart showing the transportation destination determination process performed by the sample relaying section 31a.

As shown in FIG. 15D, a "retesting rule" for determining whether measurement by the measurement unit 42 is necessary, and a "smear preparation rule" for determining whether smear preparation by the smear preparation apparatus 63 is necessary are stored in the memory 305a of the sample relaying section 31a. The controller 301a of the sample relaying section 31a refers to analysis results included in the analysis result information read in S406 in FIG. 14, and determines, for each sample, whether measurement by the measurement unit 42 and smear preparation by the smear preparation apparatus 63 are necessary, based on the retesting rule and the smear preparation rule.

When one or more samples, among the samples on the sample rack L, satisfy the retesting rule (S501: YES), the controller 301a of the sample relaying section 31a determines the measurement unit 42 as the transportation destination of the sample rack L (S502). When none of the samples, among the samples on sample rack L, satisfy the retesting rule (S501: NO) and one or more samples satisfy the smear preparation rule (S503: YES), the controller 301a of the sample relaying section 31a determines the smear preparation apparatus 63 as the transportation destination of the sample rack L (S504). When none of the samples, among the samples on the sample rack L, satisfy the retesting rule nor the smear preparation rule (S501: NO, S503: NO), the controller 301a of the sample relaying section 31a determines the collection unit 23 as the transportation destination of the sample rack L (S505).

FIG. 15C is a flow chart showing the transportation destination determination process performed by the sample relaying section 32a.

As shown in FIG. 15E, a "smear preparation rule" for determining whether smear preparation by the smear preparation apparatus 63 is necessary is stored in the memory 305a of the sample relaying section 32a. The controller 301a of the sample relaying section 32a refers to analysis results included in the analysis result information read in S406 in FIG. 14, and determines, for each sample, whether smear preparation by the smear preparation apparatus 63 is necessary, based on the smear preparation rule.

When one or more samples, among the samples on the sample rack L, satisfy the smear preparation rule (S511: YES), the controller 301a of the sample relaying section 32a determines the smear preparation apparatus 63 as the transportation destination of the sample rack L (S512). When none of the samples, among the samples on the sample rack L, satisfy the smear preparation rule (S511: NO), the controller 301a of the sample relaying section 32a determines the collection unit 23 as the transportation destination of the sample rack L (S513).

As described above, according to the present embodiment, when a sample rack L is sent onto the left table 330, it is determined whether the retesting rule and the smear preparation rule are satisfied, based on the analysis result information read from the RFID tag L1 of the sample rack L, in the transportation destination determination process. Then, the transportation destination of the sample rack L is determined. Accordingly, it becomes possible to efficiently transport samples measured by the measurement unit 41 to the measurement unit 42 and the smear preparation apparatus 63 on the downstream side, and it becomes possible to efficiently transport samples measured by the measurement unit 42 to the smear preparation apparatus 63 on the downstream side.

Further, according to the present embodiment, when the retesting rule and the smear preparation rule are not satisfied, the collection unit 23 is determined as the transportation destination of the sample rack L. Accordingly, since samples that need not be subjected to processing on the downstream side are transported to the collection unit 23, it becomes possible to more quickly transport samples that need to be subjected to processing on the downstream side, to their transportation destination.

### <Embodiment 3>

In Embodiments 1 and 2 above, the transporting units 31 to 33 are connected to each other, and a sample rack L is transported among the measurement units 41 and 42, and the smear preparation apparatus 63. In the present embodiment, a plurality of processing units are installed at separated places in a facility, and a user carries each sample rack L.

FIG. 16 is a schematic diagram showing configurations of the blood cell analyzer 61 and the smear preparation apparatus 63 of the present embodiment viewed from above. FIG. 16 shows a configuration that includes only the transporting units 31 and 33, the blood cell analyzer 61, and the smear preparation apparatus 63 from the sample processing system 1 of Embodiment 1. Further, each of the transporting units 31 and 33 in FIG. 16 includes only the components related to the measurement line (the right table 310, the rack transporter 320, and the left table 330).

As in the case of Embodiment 1, the user causes sample containers T to be held in a sample rack L as shown in FIG. 2. At this time, rack information and analysis result information as shown in FIG. 4C and FIG. 4D are written in advance in the RFID tag L1 of the sample rack L. At this time point, no analysis result of each sample ID exists in the analysis result information.

Next, the user loads the sample rack L on the right table 310 of the transporting unit 31. Accordingly, the controller 511 of the information processing unit 51 determines that a sample rack L has been sent in the measurement line (S101 in FIG. 10: YES), and performs the processing shown in FIG. 10 as in Embodiment 1. That is, the antenna A21 reads the rack information and the analysis result information from the RFID tag L1 of the sample rack L, and the measurement unit 41 performs measurement based on the read information.

It should be noted that, in the blood cell analyzer 61 of the present embodiment, after results of first tests are written in the RFID tag L1, the sample rack L is not sent out to the left table 330 from the measurement line, but instead, the processing is returned to S102 and retests are performed. Then, after the results of the retests are written in the RFID tag L1, the sample rack L is sent out to the left table 330 from the measurement line, and the measurement by the blood cell analyzer 61 is completed.

Next, the user loads the sample rack L accommodated in the left table 330 of the transporting unit 31, on the right table 310 of the transporting unit 33. Accordingly, the controller 331 of the transporting unit 33 determines that a sample rack L has been sent in the measurement line (S201 in FIG. 11A: YES), and the processing shown in FIG. 11A and the processing shown in FIG. 11B are performed as in Embodiment 1. That is, as in Embodiment 1, the antenna A23 of the transporting unit 33 reads the rack information and the analysis result information from the RFID tag L1 of the sample rack L, and the smear preparation apparatus 63 performs smear preparation based on the read information. Then, when the processing by the smear preparation apparatus 63 has been completed, the sample rack L is sent out from the measurement line to the left table 330, and the processing by the smear preparation apparatus 63 is completed.

As described above, also in the present embodiment, each of the blood cell analyzer 61 and the smear preparation apparatus 63 need not communicate with another apparatus (such as the host computer 8 in FIG. 1) in order to obtain rack information and analysis result information. Therefore, it is possible to suppress communication processing in these apparatuses from being complicated. Further, since these apparatuses need not communicate with another apparatus, it is possible to save time and costs necessary to set computers so as to allow communication therebetween.

### <Embodiment 4>

In Embodiment 1 above, a bar code label T1 is attached to each sample container T and an RFID tag L1 is attached to each sample rack L. As shown in FIG. 17A, an RFID tag T3, instead of the bar code label T1, is attached to each sample container T of the present embodiment. In the RFID tag T3, a corresponding sample ID is stored as an initial state. Further, in the present embodiment, no RFID tag L1 is attached to each sample rack L.

FIG. 17B is a schematic diagram showing a configuration of an antenna unit R according to the present embodiment viewed from above. The antenna unit R is provided in a rear portion of the preprocessing unit 22, and the bar code unit B and the antennas A11 and A12 shown in FIG. 3 are eliminated in the present embodiment.

As in the case of the bar code unit B, the antenna unit R includes two moving parts R1 provided in parallel to each other in the left-right direction. The two moving parts R1 are configured to be able to move in the left-right direction. Each moving part R1 includes two shields R11 and an antenna R12. The two shields R11 are set to their corresponding antenna R12 so as to sandwich the front face portion of the antenna R12 from the left and the right directions, and project forward further than the front face of the antenna R12 in the reading direction (downward in the drawing) of the antenna R12. Each antenna R12 is fixed to its corresponding moving part R1, and reads/writes information from/into the RFID tag T3 of a sample container T located in front of it.

Since each antenna R12 is separated from its left space and right space by the shields R11, the antenna R12 can properly read/write information from/to the RFID tag T3 of a sample container T located in front of it. Further, when the antenna R12 has performed a reading operation in the forward direction, and has been successful in reading information within a predetermined time period, it is determined that a sample container T is being held at this support position; and when the antenna R12 has not been successful in reading information within the predetermined time period, it is determined that no sample container T is being held at this support position.

It should be noted that the antenna R12 of the left moving part R1 performs reading and writing with respect to the RFID tags T3 of sample containers T held at support positions 1 to 5, and the antenna R12 of the right moving part R1 performs reading and writing with respect to the RFID tags T3 of sample containers T held at support positions 6 to 10.

Next, reading and writing with respect to an RFID tag T3 in the present embodiment will be described.

As shown in FIG. 17B, a sample rack L sent out from the loading unit 21 is located to the front of the antenna unit R. Then, the antenna unit R reads sample IDs from the RFID tags T3 of respective sample containers T held in the sample rack L, and obtains support positions of the respective sample containers T held in the sample rack L. As in the case of Embodiment 1, the preprocessing unit 22 inquires of the transportation controller 7 about the sample IDs, and obtains measurement orders and patient information from the transportation controller 7. Then, the antenna unit R writes sample information shown in FIG. 17C, into the RFID tag T3 of each sample container T.

Subsequently, when the sample rack L is transported to the rack transporter 320 of the transporting unit 31, the sample rack L is transported leftward on the rack transporter 320 by means of the belts 321a or 321b, to be located near a position P3 which is to the front of the antenna A21. Here, as shown in FIG. 17D, the antenna A21 performs reading operations simultaneously toward the support positions 1 to 10 of the sample rack L. It should be noted that, as shown in FIG. 17C, a support position as well as a sample ID, a measurement order, and patient information are written in the sample information stored in the RFID tag T3. Accordingly, even though reading operations are simultaneously performed toward the support positions 1 to 10, it is possible to know which read sample information corresponds to a sample of which support position, based on the support position included in the sample information read by the antenna A21.

Subsequently, a sample container T is located at the position P2, and each sample held in the sample rack L is sequentially measured. When the measurement of a sample has been completed and its sample container T is returned to its original support position, an antenna A41 writes analysis result information as shown in FIG. 17E. The analysis result information includes a sample ID, a support position, and an analysis result based on measurement data obtained by the measurement unit 41. As in the case of the antennas R12 of the antenna unit R, the antenna A41 is provided with two shields 323a. The two shields 323a allow the antenna A41 to properly write analysis result information in the RFID tag T3 of the sample container T located in front of it.

Next, when the sample rack L is transported to the downstream side and transported to the rack transporter 320 of the transporting unit 32, the sample rack L is located near the antenna A22, and the antenna A22 reads sample information and analysis result information of all the samples held in the sample rack L, as in the case of the transporting unit 31. Then, the measurement unit 42 performs measurement based on the read analysis result information, and the antenna A41 provided on the rack transporter 320 of the transporting unit 32 writes corresponding analysis result information into the RFID tag T3 of each sample container T.

It should be noted that, in the present embodiment, the antenna A13 provided in the collection unit 23 is also provided with shields as in the case of the antenna A41 shown in FIG. 17D. Thus, the antenna A13 reads an analysis result from the RFID tag T3 of each sample container T.

As described above, also in the present embodiment, the blood cell analyzers 61 and 62, and the smear preparation apparatus 63 need not communicate with another apparatus (such as the host computer 8) in order to obtain sample information and analysis result information. Accordingly, it is possible to obtain similar effects as those of Embodiment 1.

Embodiments of the present invention have been described above. However, embodiments of the present invention are not limited thereto.

For example, the above embodiments have illustrated a case where blood is measured in the measurement units 41 and 42. However, urine may be measured by the measurement units 41 and 42. That is, it is possible to apply the present invention to a sample processing system that includes measurement units for measuring urine, and further, it is possible to apply the present invention to a clinical sample processing system that includes measurement units for measuring other clinical samples.

Further, although in Embodiments 1, 2, and 4 above, the sample processing system 1 is communicably connected to the host computer 8, the present invention is not limited thereto. The sample processing system 1 may not be connected to the host computer 8. In this case, the blood cell analyzer 61 for performing first tests may perform measurement for predetermined measurement items. Alternatively, rack information (Embodiments 1 and 2) and sample information (Embodiment 4) which are written in the preprocessing unit 22 may include predetermined measurement items.

Further, in Embodiments 1 to 3, each antenna provided in the rack transporter 320 performs both of reading and writing with respect to an RFID tag L1. However, the present invention is not limited thereto. The rack transporter 320 may be provided with separate antennas which respectively perform reading and writing. Further, in Embodiment 4, the rack transporter 320 is provided with separate antennas which respectively perform reading and writing. However, the present invention is not limited thereto. The rack transporter 320 may be provided with one antenna that performs both of reading and writing. In this case, the antenna is provided with shields so as to be able to perform writing onto the RFID tag T3 of each sample container T individually, as in the case of the antenna A41 shown in FIG. 17D.

In addition to the above, various modifications of the embodiment of the present invention may be made as appropriate without departing from the scope of the technical idea defined by the claims.

## Claims

1. A sample processing apparatus comprising:
a reader configured to read, from a storage medium attached to a sample container containing a sample or a sample rack supporting the sample container, an analysis value which has been generated by a sample analyzer analyzing the sample for a predetermined analysis item;
a sample processing section which comprises an operating mechanism configured to perform an operation for processing the sample, and
a controller configured to control the operating mechanism of the sample processing section so as to perform processing on the sample based on the analysis value read by the reader.

2. The sample processing apparatus of claim 1, wherein
the sample processing section is a sample analyzing section configured to analyze the sample, and
the controller controls the operating mechanism of the sample analyzing section so as to analyze the sample for an analysis item according to the analysis value read by the reader.

3. The sample processing apparatus of claim 2, further comprising
a writer configured to write a result of a sample analysis performed by the sample analyzing section into the storage medium.

4. The sample processing apparatus of any one of claims 1 to 3, wherein
the sample processing section is a smear preparation section configured to prepare a smear by smearing a sample, and
when the analysis value read by the reader satisfies a predetermined condition, the controller controls the operating mechanism so as to prepare the smear.

5. The sample processing apparatus of claim 4, wherein
the controller controls the operating mechanism of the smear preparation section so as to prepare the smear on a condition according to the analysis value read by the reader.

6. The sample processing apparatus of any one of claims 1 to 5, wherein
the sample is a biospecimen collected from a subject.

7. The sample processing apparatus of any one of claims 1 to 6, further comprising:
the sample analyzer;
a writer configured to write the analysis value generated by the sample analyzer into the storage medium; and
a transporting apparatus configured to transport the sample container or the sample rack to the sample processing section, the analysis value having been written by the writer into the storage medium attached to the sample container or the sample rack, wherein
the reader reads the analysis value from the storage medium attached to the sample container or the sample rack transported by the transporting apparatus.

8. The sample processing apparatus of claim 7, wherein
the storage medium is attached to the sample rack,
the sample rack is capable of supporting a plurality of sample containers,
the sample processing apparatus further comprises a detector configured to detect a support position of each sample container in the sample rack, and
the writer writes, into the storage medium, an analysis value of a sample in each sample container in association with a support position of each sample container detected by the detector, respectively.

9. The sample processing apparatus of claim 7 or 8, wherein
the transporting apparatus is formed by a first transporting unit corresponding to the sample analyzer and a second transporting unit corresponding to the sample processing section, and
the reader is arranged in the second transporting unit, and reads the analysis value from the storage medium attached to the sample container or the sample rack that has been transported from the first transporting unit to the second transporting unit.

10. The sample processing apparatus of any one of claims 7 to 9, further comprising
a second sample processing section comprising an operating mechanism configured to perform an operation for processing the sample transported by the transporting apparatus, wherein
the controller determines, from among the sample processing section and the second sample processing section, a transportation destination for the sample analyzed by the sample analyzer, based on the analysis value read by the reader, and controls the transporting apparatus so as to transport the sample to the determined transportation destination.

11. The sample processing apparatus of any one of claims 1 to 10, wherein
the storage medium is an electronic tag communicable with the reader, and
the reader is configured to read the analysis value from the electronic tag, by using radio waves.

12. A sample processing system comprising:
a sample analyzing section configured to analyze a sample to generate an analysis value for a predetermined analysis item;
a transporting apparatus configured to transport a sample container containing the sample analyzed by the sample analyzing section or a sample rack supporting the sample container;
a first to an nth sample processing sections each comprising an operating mechanism configured to perform an operation for processing the sample transported by the transporting apparatus, and arranged downstream to the sample analyzing section in a sample transporting direction of the transporting apparatus;
a writer configured to write the analysis value generated by the sample analyzing section into a storage medium attached to the sample container containing the sample analyzed by the sample analyzing section or the sample rack supporting the sample container;
a reader configured to read the analysis value written by the writer from the storage medium; and
a transportation controller configured to determine, from among the first to the nth sample processing sections, a transportation destination for the sample analyzed by the sample analyzing section, based on the analysis value read by the reader, and configured to control a transporting operation of the transporting apparatus so as to transport the sample to the determined transportation destination.

13. The sample processing system of claim 12, wherein
the transportation controller stores a rule set for determining whether it is necessary to perform sample processing in each of the first to the nth sample processing sections, and determines the transportation destination for the sample, based on the analysis value read by the reader and the rule set.

14. The sample processing system of claim 12 or 13, further comprising
a sample collection section configured to collect a sample for which processing has been completed, wherein
when it is not necessary to perform processing on the sample in any of the first to the nth sample processing sections, the transportation controller controls the transporting operation of the transporting apparatus so as to transport the sample to the sample collection section.

15. A sample processing method to be used in a sample processing system which includes a sample analyzer and at least one sample processing apparatus, the method comprising:
writing an analysis value generated by the sample analyzer analyzing a sample for a predetermined analysis item, into a storage medium attached to a sample container containing the sample or a sample rack supporting the sample container;
reading the analysis value from the storage medium in the sample processing apparatus; and
performing processing on the sample by using the sample processing apparatus, based on the read analysis value.
